# EUROPEAN PATENT APPLICATION

(11) **EP 4 059 962 A1**
(43) Date of publication of application: **21.09.2022**
(21) Application number: 20882932.5
(22) Date of filing: 30.10.2020
(51) Int. Cl.: C07K 16/28, C12N 15/13, C12N 15/63, C12N 5/10, A61K 39/395, A61P 35/00

(54) **PD-L1 BINDING MOLECULE**

(30) Priority: 30.10.2019 CN 201911044297; 31.07.2020 CN 202010765530
(71) Applicant: Sanyou Biopharmaceuticals Co., Ltd., Shanghai 200233 (CN)
(72) Inventor: KONG, Chao, Shanghai 200233 (CN); LANG, Guojun, Shanghai 200233 (CN); WU, Qi, Shanghai 200233 (CN); LIU, Chanjuan, Shanghai 200233 (CN); YAN, Run, Shanghai 200233 (CN); YAN, Xintian, Shanghai 200233 (CN); TAN, Lunmei, Shanghai 200233 (CN)
(74) Representative: Meissner Bolte Partnerschaft mbB
(86) International application number: PCT/CN2020/125301
(87) International publication number: WO 2021/083335

(57) **Abstract**

The present application provides an isolated PD-L1 binding molecule, specifically, an isolated PD-L1 single-domain antibody or an antigen-binding fragment thereof. The present invention also provides a nucleic acid encoding the isolated PD-L1 binding molecule, an expression vector or host cell comprising the nucleic acid, and a pharmaceutical composition or kit comprising the PD-L1 binding molecule.

SEQUENCE LISTING

<110>  SANYOU BIOPHARMACEUTICALS CO., LTD.

<120>  PD-L1 BINDING MOLECULE

<130>  IEC196121PCT

<150>  CN201911044297.0
<151>  2019-10-30

<150>  CN202010765530.0
<151>  2020-07-31

<160>  65

<170>  PatentIn version 3.5

<210>  1
<211>  10
<212>  PRT
<213>  Artificial Sequence

<220>
<223>  CDR1

<400>  1

Arg Thr Asp Ser Asn Ile Asn Gly Met His
1               5                   10


<210>  2
<211>  9
<212>  PRT
<213>  Artificial Sequence

<220>
<223>  CDR2

<400>  2

Thr Ile Phe Ile Asp Gly Asn Thr Ile
1               5


<210>  3
<211>  8
<212>  PRT
<213>  Artificial Sequence

<220>
<223>  CDR3

<400>  3

Asp Val Ser Gly Tyr Gly Arg Ala
1               5

<210>  4
<211>  10
<212>  PRT
<213>  Artificial Sequence

<220>
<223>  CDR1

<400>  4

Arg Thr Asp Ser Asn Ile His Gly Met His
1               5                   10


<210>  5
<211>  9
<212>  PRT
<213>  Artificial Sequence

<220>
<223>  CDR2

<400>  5

Thr Ile Phe Ile Asp Leu Asn Thr Ile
1               5


<210>  6
<211>  10
<212>  PRT
<213>  Artificial Sequence

<220>
<223>  CDR1

<400>  6

Arg Thr Asp Ser Asn Ile Phe Gly Met His
1               5                   10


<210>  7
<211>  9
<212>  PRT
<213>  Artificial Sequence

<220>
<223>  CDR2

<400>  7

Thr Ile Phe Ile Asp Ala Asn Thr Ile
1               5

<210>  8
<211>  9
<212>  PRT
<213>  Artificial Sequence

<220>
<223>  CDR2

<400>  8

Thr Ile Phe Ile Asp Gly Asn Thr Leu
1               5


<210>  9
<211>  8
<212>  PRT
<213>  Artificial Sequence

<220>
<223>  CDR3

<400>  9

Asp Val Ser Gly Tyr Gly Arg Tyr
1               5


<210>  10
<211>  10
<212>  PRT
<213>  Artificial Sequence

<220>
<223>  CDR1

<400>  10

Arg Thr Asp Ser Asn Ile Asn Ser Met His
1               5                   10


<210>  11
<211>  9
<212>  PRT
<213>  Artificial Sequence

<220>
<223>  CDR2

<400>  11

Thr Trp Phe Ile Asp Gly Asn Thr Ile
1               5


<210>  12
<211>  8

<212>  PRT
<213>  Artificial Sequence

<220>
<223>  CDR3

<400>  12

Asp Glu Trp Met Tyr Gly Arg Ala
1               5


<210>  13
<211>  10
<212>  PRT
<213>  Artificial Sequence

<220>
<223>  CDR1

<400>  13

Arg Thr Asp Arg Asn Ile Asn Thr Met His
1               5                   10


<210>  14
<211>  10
<212>  PRT
<213>  Artificial Sequence

<220>
<223>  CDR1

<400>  14

Arg Thr Tyr Ser Asn Ile Tyr Gly Met His
1               5                   10


<210>  15
<211>  9
<212>  PRT
<213>  Artificial Sequence

<220>
<223>  CDR2

<400>  15

Thr Ile Phe Ile Gly Ser Asn Thr Ile
1               5


<210>  16
<211>  10
<212>  PRT
<213>  Artificial Sequence

<220>
<223>  CDR1

<400>  16

Arg Thr Gly Ser Asn Ile Tyr Gly Met His
1               5                   10


<210>  17
<211>  9
<212>  PRT
<213>  Artificial Sequence

<220>
<223>  CDR2

<400>  17

Thr Ile Phe Ile Asp Trp Asn Thr Ile
1               5


<210>  18
<211>  10
<212>  PRT
<213>  Artificial Sequence

<220>
<223>  CDR1

<400>  18

Arg Thr Phe Ser Asn Ile Phe Gly Met His
1               5                   10


<210>  19
<211>  9
<212>  PRT
<213>  Artificial Sequence

<220>
<223>  CDR2

<400>  19

Thr Ile Phe Ile Thr Gly Asn Thr Ile
1               5


<210>  20
<211>  10
<212>  PRT
<213>  Artificial Sequence

<220>

<223>  CDR1

<400>  20

Arg Thr Leu Ser Asn Ile Arg Gly Met His
1               5                   10


<210>  21
<211>  10
<212>  PRT
<213>  Artificial Sequence

<220>
<223>  CDR1

<400>  21

Arg Arg Trp Ser Asn Ile His Gly Met His
1               5                   10


<210>  22
<211>  9
<212>  PRT
<213>  Artificial Sequence

<220>
<223>  CDR2

<400>  22

Ser Arg Phe Ile Thr Gly Asn Thr Ile
1               5


<210>  23
<211>  8
<212>  PRT
<213>  Artificial Sequence

<220>
<223>  CDR3

<400>  23

Asp Val Arg Gly Tyr Gly Arg Ala
1               5


<210>  24
<211>  10
<212>  PRT
<213>  Artificial Sequence

<220>
<223>  CDR1

<400>  24

Pro Thr Ala Ser Asn Ile His Gly Met His
1               5                   10


<210>  25
<211>  9
<212>  PRT
<213>  Artificial Sequence

<220>
<223>  CDR2

<400>  25

Met Ile Phe Ile Thr Gly Leu Thr Ile
1               5


<210>  26
<211>  10
<212>  PRT
<213>  Artificial Sequence

<220>
<223>  CDR1

<400>  26

Arg Thr Ala Ser Asn Ala Met Gly Met His
1               5                   10


<210>  27
<211>  9
<212>  PRT
<213>  Artificial Sequence

<220>
<223>  CDR2

<400>  27

Met Ile Phe Arg Asp Gly Asn Thr Ile
1               5


<210>  28
<211>  10
<212>  PRT
<213>  Artificial Sequence

<220>
<223>  CDR1

<400>  28

Arg Thr Ala Gly Asn Ile His Gly Met His
1               5                   10

<210>  29
<211>  9
<212>  PRT
<213>  Artificial Sequence

<220>
<223>  CDR2

<400>  29

Ser Ile Phe Ile Thr Gly Asn Thr Ile
1               5

<210>  30
<211>  10
<212>  PRT
<213>  Artificial Sequence

<220>
<223>  CDR1

<400>  30

Arg Thr Ala Gly Tyr Ile His Gly Met His
1               5                   10

<210>  31
<211>  9
<212>  PRT
<213>  Artificial Sequence

<220>
<223>  CDR2

<400>  31

Met Ile Phe Gly Trp Gly Asn Thr Ile
1               5

<210>  32
<211>  10
<212>  PRT
<213>  Artificial Sequence

<220>
<223>  CDR1

<400>  32

Gly Phe Thr Phe Ser Arg Arg Cys Lys Ala
1               5                   10

<210>  33
<211>  10
<212>  PRT
<213>  Artificial Sequence

<220>
<223>  CDR2

<400>  33

Lys Ile Leu Thr Thr Ser Gly Ser Thr Tyr
1               5                   10


<210>  34
<211>  19
<212>  PRT
<213>  Artificial Sequence

<220>
<223>  CDR3

<400>  34

Asp Ser Phe Glu Asp Pro Thr Cys Thr Leu Val Thr Ser Ser Gly Ala
1               5                   10                  15


Phe Gln Tyr




<210>  35
<211>  128
<212>  PRT
<213>  Artificial Sequence

<220>
<223>  VH

<400>  35

Gln Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Arg Arg
            20                  25                  30


Cys Lys Ala Trp Phe Arg Gln Ala Pro Gly Lys Glu Arg Glu Arg Val
        35                  40                  45


Ala Lys Ile Leu Thr Thr Ser Gly Ser Thr Tyr Leu Ala Asp Ser Val
    50                  55                  60

Lys Gly Arg Phe Thr Ile Ser Arg Asp Asn Ser Lys Asn Thr Val Tyr
65                  70                  75                  80

Leu Gln Met Asn Ser Leu Arg Ala Glu Asp Thr Ala Val Tyr Tyr Cys
                85                  90                  95

Ala Ala Asp Ser Phe Glu Asp Pro Thr Cys Thr Leu Val Thr Ser Ser
            100                 105                 110

Gly Ala Phe Gln Tyr Trp Gly Gln Gly Thr Leu Val Thr Val Ser Ser
        115                 120                 125

<210>  36
<211>  116
<212>  PRT
<213>  Artificial Sequence

<220>
<223>  VH

<400>  36

Glu Val Gln Val Gln Glu Ser Gly Gly Gly Leu Val Gln Ala Gly Gly
1               5                   10                  15

Ser Leu Arg Leu Ser Cys Ala His Ser Arg Thr Asp Ser Asn Ile Asn
            20                  25                  30

Gly Met His Trp Tyr Arg Gln Ala Pro Gly Lys Gln Arg Asp Trp Val
        35                  40                  45

Gly Thr Ile Phe Ile Asp Gly Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Lys Pro Glu Asp Thr Ala Val Tyr Phe Cys Ala
                85                  90                  95

Ala Asp Val Ser Gly Tyr Gly Arg Ala Trp Gly Gln Gly Thr Gln Val
            100                 105                 110

Thr Val Ser Ser
        115

<210>  37
<211>  116
<212>  PRT
<213>  Artificial Sequence

<220>
<223>  VH

<400>  37

Glu Val Gln Leu Val Glu Ser Gly Gly Gly Leu Val Gln Pro Gly Gly
1               5                   10                  15

Ser Leu Arg Leu Ser Cys Ala Ala Ser Arg Thr Asp Ser Asn Ile Asn
            20                  25                  30

Gly Met His Trp Tyr Arg Gln Ala Pro Gly Lys Gly Arg Glu Trp Val
        35                  40                  45

Gly Thr Ile Phe Ile Asp Gly Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115


<210>  38
<211>  116
<212>  PRT
<213>  Artificial Sequence

<220>
<223>  VH

<400>  38

Glu Val Gln Leu Val Glu Ser Gly Gly Gly Leu Val Gln Pro Gly Gly
1               5                   10                  15

Ser Leu Arg Leu Ser Cys Ala His Ser Arg Thr Asp Ser Asn Ile Asn
            20                  25                  30

Gly Met His Trp Tyr Arg Gln Ala Pro Gly Lys Gly Arg Glu Trp Val
        35                  40                  45

Gly Thr Ile Phe Ile Asp Gly Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115

<210>  39
<211>  116
<212>  PRT
<213>  Artificial Sequence

<220>
<223>  VH

<400>  39

Glu Val Gln Leu Val Glu Ser Gly Gly Gly Leu Val Gln Pro Gly Gly
1               5                   10                  15

Ser Leu Arg Leu Ser Cys Ala His Ser Arg Thr Asp Ser Asn Ile His
            20                  25                  30

Gly Met His Trp Tyr Arg Gln Ala Pro Gly Lys Gly Arg Glu Trp Val
        35                  40                  45

Gly Thr Ile Phe Ile Asp Leu Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115

<210>  40
<211>  116
<212>  PRT
<213>  Artificial Sequence

<220>
<223>  VH

<400>  40

Glu Val Gln Leu Val Glu Ser Gly Gly Gly Leu Val Gln Pro Gly Gly
1               5                   10                  15

Ser Leu Arg Leu Ser Cys Ala His Ser Arg Thr Asp Ser Asn Ile Phe
            20                  25                  30

Gly Met His Trp Tyr Arg Gln Ala Pro Gly Lys Gly Arg Glu Trp Val
        35                  40                  45

Gly Thr Ile Phe Ile Asp Ala Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115

<210>  41
<211>  116
<212>  PRT
<213>  Artificial Sequence

<220>
<223>  VH

<400>  41

Glu Val Gln Leu Val Glu Ser Gly Gly Gly Leu Val Gln Pro Gly Gly
1               5                   10                  15

Ser Leu Arg Leu Ser Cys Ala His Ser Arg Thr Asp Ser Asn Ile Asn
            20                  25                  30

Gly Met His Trp Tyr Arg Gln Ala Pro Gly Lys Gly Arg Glu Trp Val
        35                  40                  45

Gly Thr Ile Phe Ile Asp Gly Asn Thr Leu Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Tyr Trp Gly Gln Gly Thr Thr Val
            100                 105                 110

Thr Val Ser Ser
        115


<210>  42
<211>  116
<212>  PRT
<213>  Artificial Sequence

<220>
<223>  VH

<400>  42

Glu Val Gln Leu Val Glu Ser Gly Gly Gly Leu Val Gln Pro Gly Gly
1               5                   10                  15

Ser Leu Arg Leu Ser Cys Ala His Ser Arg Thr Asp Ser Asn Ile Asn
            20                  25                  30

Ser Met His Trp Tyr Arg Gln Ala Pro Gly Lys Gly Arg Glu Trp Val
        35                  40                  45

Gly Thr Trp Phe Ile Asp Gly Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Glu Trp Met Tyr Gly Arg Ala Trp Gly Gln Gly Thr Thr Val
            100                 105                 110

Thr Val Ser Ser
        115

<210>  43
<211>  116
<212>  PRT
<213>  Artificial Sequence

<220>
<223>  VH

<400>  43

Glu Val Gln Leu Val Glu Ser Gly Gly Gly Leu Val Gln Pro Gly Gly
1               5                   10                  15

Ser Leu Arg Leu Ser Cys Ala Ala Ser Arg Thr Asp Arg Asn Ile Asn
            20                  25                  30

Thr Met His Trp Tyr Arg Gln Ala Pro Gly Lys Gly Arg Glu Trp Val
        35                  40                  45

Gly Thr Ile Phe Ile Asp Leu Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115

<210>  44
<211>  116
<212>  PRT
<213>  Artificial Sequence

<220>
<223>  VH

<400>  44

Glu Val Gln Leu Val Glu Ser Gly Gly Gly Leu Val Gln Pro Gly Gly
1               5                   10                  15

Ser Leu Arg Leu Ser Cys Ala Ala Ser Arg Thr Tyr Ser Asn Ile Tyr
            20                  25                  30

Gly Met His Trp Tyr Arg Gln Ala Pro Gly Lys Gly Arg Glu Trp Val
        35                  40                  45

Gly Thr Ile Phe Ile Gly Ser Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115

<210>  45
<211>  116
<212>  PRT
<213>  Artificial Sequence

<220>
<223>  VH

<400>  45

Glu Val Gln Leu Val Glu Ser Gly Gly Gly Leu Val Gln Pro Gly Gly
1               5                   10                  15

Ser Leu Arg Leu Ser Cys Ala Ala Ser Arg Thr Gly Ser Asn Ile Tyr
            20                  25                  30

Gly Met His Trp Tyr Arg Gln Ala Pro Gly Lys Gly Arg Glu Trp Val
        35                  40                  45

Gly Thr Ile Phe Ile Asp Trp Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115

<210>  46
<211>  116
<212>  PRT
<213>  Artificial Sequence

<220>
<223>  VH

<400>  46

Glu Val Gln Leu Val Glu Ser Gly Gly Gly Leu Val Gln Pro Gly Gly
1               5                   10                  15

Ser Leu Arg Leu Ser Cys Ala Ala Ser Arg Thr Phe Ser Asn Ile Phe
            20                  25                  30

Gly Met His Trp Tyr Arg Gln Ala Pro Gly Lys Gly Arg Glu Trp Val
        35                  40                  45

Gly Thr Ile Phe Ile Thr Gly Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115

<210>  47
<211>  116
<212>  PRT
<213>  Artificial Sequence

<220>
<223>  VH

<400>  47

Glu Val Gln Leu Val Glu Ser Gly Gly Gly Leu Val Gln Pro Gly Gly
1               5                   10                  15

Ser Leu Arg Leu Ser Cys Ala Ala Ser Arg Thr Leu Ser Asn Ile Arg
            20                  25                  30

Gly Met His Trp Tyr Arg Gln Ala Pro Gly Lys Gly Arg Glu Trp Val
        35                  40                  45

Gly Thr Ile Phe Ile Asp Leu Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115

<210>  48
<211>  116
<212>  PRT
<213>  Artificial Sequence

<220>
<223>  VH

<400>  48

Glu Val Gln Leu Val Glu Ser Gly Gly Gly Leu Val Gln Pro Gly Gly
1               5                   10                  15

Ser Leu Arg Leu Ser Cys Ala Ala Ser Arg Arg Trp Ser Asn Ile His
            20                  25                  30

Gly Met His Trp Tyr Arg Gln Ala Pro Gly Lys Gly Arg Glu Trp Val
        35                  40                  45

Gly Ser Arg Phe Ile Thr Gly Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Arg Gly Tyr Gly Arg Ala Trp Gly Gln Gly Thr Thr Val
            100                 105                 110

Thr Val Ser Ser
        115

<210>  49
<211>  116
<212>  PRT
<213>  Artificial Sequence

<220>
<223>  VH

<400>  49

Glu Val Gln Leu Val Glu Ser Gly Gly Gly Leu Val Gln Pro Gly Gly
1               5                   10                  15

Ser Leu Arg Leu Ser Cys Ala Ala Ser Pro Thr Ala Ser Asn Ile His
            20                  25                  30

Gly Met His Trp Tyr Arg Gln Ala Pro Gly Lys Gly Arg Glu Trp Val
        35                  40                  45

Gly Met Ile Phe Ile Thr Gly Leu Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Ser Gly Tyr Gly Arg Ala Trp Gly Gln Gly Thr Thr Val
            100                 105                 110

Thr Val Ser Ser
        115

<210> 50
<211> 116
<212> PRT
<213> Artificial Sequence

<220>
<223> VH

<400> 50

Glu Val Gln Leu Val Glu Ser Gly Gly Gly Leu Val Gln Pro Gly Gly
1               5                   10                  15

Ser Leu Arg Leu Ser Cys Ala Ala Ser Arg Thr Ala Ser Asn Ala Met
            20                  25                  30

Gly Met His Trp Tyr Arg Gln Ala Pro Gly Lys Gly Arg Glu Trp Val
        35                  40                  45

Gly Met Ile Phe Arg Asp Gly Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Arg Gly Tyr Gly Arg Ala Trp Gly Gln Gly Thr Thr Val
            100                 105                 110

Thr Val Ser Ser
        115

<210>  51
<211>  116
<212>  PRT
<213>  Artificial Sequence

<220>
<223>  VH

<400>  51

Glu Val Gln Leu Val Glu Ser Gly Gly Gly Leu Val Gln Pro Gly Gly
1               5                   10                  15

Ser Leu Arg Leu Ser Cys Ala Ala Ser Arg Thr Ala Gly Asn Ile His
            20                  25                  30

Gly Met His Trp Tyr Arg Gln Ala Pro Gly Lys Gly Arg Glu Trp Val
        35                  40                  45

Gly Ser Ile Phe Ile Thr Gly Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Arg Gly Tyr Gly Arg Ala Trp Gly Gln Gly Thr Thr Val
            100                 105                 110

Thr Val Ser Ser
        115

<210>  52
<211>  116
<212>  PRT
<213>  Artificial Sequence

<220>

<223>  VH

<400>  52

Glu Val Gln Leu Val Glu Ser Gly Gly Gly Leu Val Gln Pro Gly Gly
1               5                   10                  15

Ser Leu Arg Leu Ser Cys Ala Ala Ser Arg Thr Ala Gly Tyr Ile His
            20                  25                  30

Gly Met His Trp Tyr Arg Gln Ala Pro Gly Lys Gly Arg Glu Trp Val
        35                  40                  45

Gly Met Ile Phe Gly Trp Gly Asn Thr Ile Val Thr Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Thr Leu Arg Ala Glu Asp Thr Ala Val Tyr Tyr Cys Ala
                85                  90                  95

Ala Asp Val Arg Gly Tyr Gly Arg Ala Trp Gly Gln Gly Thr Thr Val
            100                 105                 110

Thr Val Ser Ser
        115

<210>  53
<211>  25
<212>  PRT
<213>  Artificial Sequence

<220>
<223>  FR1

<400>  53

Glu Val Gln Val Gln Glu Ser Gly Gly Gly Leu Val Gln Ala Gly Gly
1               5                   10                  15

Ser Leu Arg Leu Ser Cys Ala His Ser
            20                  25

<210>  54
<211>  14
<212>  PRT
<213>  Artificial Sequence

<220>
<223>  FR2

<400>  54

Trp Tyr Arg Gln Ala Pro Gly Lys Gln Arg Asp Trp Val Gly
1               5                   10


<210>  55
<211>  39
<212>  PRT
<213>  Artificial Sequence

<220>
<223>  FR3

<400>  55

Val Thr Asp Ser Val Lys Gly Arg Phe Thr Ile Ser Arg Asp Asn Ala
1               5                   10                  15


Lys Asn Thr Leu Tyr Leu Gln Met Asn Thr Leu Lys Pro Glu Asp Thr
            20                  25                  30


Ala Val Tyr Phe Cys Ala Ala
        35


<210>  56
<211>  11
<212>  PRT
<213>  Artificial Sequence

<220>
<223>  FR4

<400>  56

Trp Gly Gln Gly Thr Gln Val Thr Val Ser Ser
1               5                   10


<210>  57
<211>  25
<212>  PRT
<213>  Artificial Sequence

<220>
<223>  FR1

<400>  57

<210>  58
<211>  14
<212>  PRT
<213>  Artificial Sequence

<220>
<223>  FR2

<400>  58

Trp Tyr Arg Gln Ala Pro Gly Lys Gly Arg Glu Trp Val Gly
1               5                   10

<210>  59
<211>  39
<212>  PRT
<213>  Artificial Sequence

<220>
<223>  FR3

<400>  59

Val Thr Asp Ser Val Lys Gly Arg Phe Thr Ile Ser Arg Asp Asn Ala
1               5                   10                  15

Lys Asn Thr Leu Tyr Leu Gln Met Asn Thr Leu Arg Ala Glu Asp Thr
            20                  25                  30

Ala Val Tyr Tyr Cys Ala Ala
        35

<210>  60
<211>  11
<212>  PRT
<213>  Artificial Sequence

<220>
<223>  FR4

<400>  60

Trp Gly Gln Gly Thr Thr Val Thr Val Ser Ser
1               5                   10

<210>  61
<211>  25

<212>  PRT
<213>  Artificial Sequence

<220>
<223>  FR1

<400>  61

Glu Val Gln Leu Val Glu Ser Gly Gly Gly Leu Val Gln Pro Gly Gly
1               5                   10                  15

Ser Leu Arg Leu Ser Cys Ala His Ser
            20                  25

<210>  62
<211>  25
<212>  PRT
<213>  Artificial Sequence

<220>
<223>  FR1

<400>  62

Gln Val Gln Leu Val Glu Ser Gly Gly Gly Leu Val Gln Pro Gly Gly
1               5                   10                  15

Ser Leu Arg Leu Ser Cys Ala Ala Ser
            20                  25

<210>  63
<211>  14
<212>  PRT
<213>  Artificial Sequence

<220>
<223>  FR2

<400>  63

Trp Phe Arg Gln Ala Pro Gly Lys Glu Arg Glu Arg Val Ala
1               5                   10

<210>  64
<211>  39
<212>  PRT
<213>  Artificial Sequence

<220>
<223>  FR3

<400>  64

Leu Ala Asp Ser Val Lys Gly Arg Phe Thr Ile Ser Arg Asp Asn Ser
1               5                   10                  15

Lys Asn Thr Val Tyr Leu Gln Met Asn Ser Leu Arg Ala Glu Asp Thr
            20                  25                  30

Ala Val Tyr Tyr Cys Ala Ala
        35

<210>  65
<211>  11
<212>  PRT
<213>  Artificial Sequence

<220>
<223>  FR4

<400>  65

Trp Gly Gln Gly Thr Leu Val Thr Val Ser Ser
1               5                   10

## Description

### Claim of Priority

The present application claims the benefits of Chinese invention patent application No. 201911044297.0 filed on October 30, 2019 and Chinese invention patent application No. 202010765530.0 filed on July 31, 2020, the content of which is fully incorporated herein by reference.

### Technical Field

The present application belongs to the technical field of biology, and in general, relates to antibodies. More specifically, the present application relates to a binding molecule (e.g., a single-domain antibody) that specifically recognizes PD-L1, a method for preparing same and the use thereof.

### Background of the Invention

Immune checkpoints are regulatory molecules that play an inhibitory role in the immune system, including CTLA-4, PD-1, LAG-3, TIM-3, etc. At present, there are a plurality of antibody drugs on the basis of the two immune checkpoints CTLA-4 and PD-1 on the market. PD-1 (programmed death 1), an important immunosuppressive molecule, was originally cloned from apoptotic mouse T cell hybridoma 2B4.11. Immunoregulation targeting PD-1 is of great significance in the anti-tumor, anti-infection, anti-autoimmune diseases and survival of organ transplantation.

The main ligands of PD-1 are PD-L1 and PD-L2, of which PD-L1 plays an important role in mediating tumor cell escape and is highly expressed in tumor cells and some antigen-presenting cells, with the expression level capable of being induced by various cytokines such as IFN-γ and TGF-β. In a tumor microenvironment, the upregulation of PD-L1 expression can directly inhibit the anti-tumor response of T cells and mediate the immune escape of tumor cells via a PD-1 signaling pathway.

A single-domain antibody (referred to as sdAb for short) is an antibody comprising a single variable domain of heavy chain of an antibody. Similar to an IgG antibody, the single-domain antibody can selectively bind to a specific antigen, but has a molecular weight much smaller than that of the IgG antibody. At present, the first single-domain antibody is engineered from a heavy chain antibody found in camelids (Hamers-Casterman C, Atarhouch T, Muyldermans S, Robinson G, Hamers C, Songa EB, Bendahman N, Hamers R (1993) Naturally Occurring Antibodies Devoid of Light Chains. Nature 363(6428):446-448); and the heavy chain antibody found in camelids is also referred to as a VHH fragment. At present, most of the research on single-domain antibodies are based on heavy chain variable domains.

Single-domain antibodies have many advantages. For example, they have quite high solubility, good thermal stability and good tissue penetration. Due to the presence of an intramolecular disulfide bond, some single-domain antibodies can also be resistant to the degradation by papains, etc. In addition, single-domain antibodies can be produced, in a quite high expression level, in a plurality of expression hosts such as yeast, plant and mammalian cells, which endows them with very great cost effectiveness (Harmsen MM, De Haard HJ (2007) Properties, Production, and Applications of Camelid Single-domain Antibody Fragments. Appl Microbiol Biotechnol 77(1):13-22). Single-domain antibodies have good application prospects in various biotechnology and medical fields due to their numerous advantages. At present, the first single-domain antibody drug of Ablynx has been approved for marketing.

Up to now, there are not too many antibody drugs targeting PD-L1 on the market. Therefore, there is still a need to develop new binding molecules (e.g., single-domain antibodies) that specifically recognize PD-L1 for cancer immunotherapy, making them to have lower toxicity, lower side effects and better clinical efficacy.

### Brief Summary of the Invention

The objective of the present invention is to provide a new PD-L1 binding molecule, specifically, to provide a new single-domain antibody that specifically recognizes PD-L1. Compared with the existing PD-L1 binding molecules, the PD-L1 binding molecule of the present invention has lower toxicity and side effects and better clinical efficacy, and can treat cancers more effectively.

In general, the present invention provides a binding molecule that specifically recognizes PD-L1, specifically, a single-domain antibody that specifically recognizes PD-L1. The single-domain antibody is also referred to hereinafter as a PD-L1 single-domain antibody, a PD-L1 antibody in a nanobody format, a PD-L1 nanobody or a VHH antibody of PD-L1, and the above-mentioned terms can be used interchangeably. The present application also provides a method for constructing and screening the PD-L1 binding molecule, a nucleic acid molecule encoding the PD-L1 binding molecule, a vector and a host cell for expressing the PD-L1 binding molecule, and a composition or a kit comprising the PD-L1 binding molecule. The PD-L1 binding molecule of the present application can treat various cancers by modulating the immune system, and can thus be used to prepare an agent for treating cancers.

Specifically, the present invention provides a PD-L1 antibody in a nanobody format and a humanized modified or an affinity-matured molecule derived therefrom. While the advantages of the PD-L1 antibody, such as high affinity and low molecular weight are maintained, immunogenicity modification is performed thereon to improve the druggability, making same to have great therapeutic advantages.

In some aspects, the present invention provides an isolated PD-L1 binding molecule, which specifically binds to PD-L1 and comprises a heavy chain variable region, wherein the heavy chain variable region comprises:
(i) a CDR1 comprising the amino acid sequence of SEQ ID NO: 1;
   a CDR1 comprising an amino acid sequence having at least 80%, 85%, 90%, 95% or 99% identity to SEQ ID NO: 1; or
   a CDR1 comprising an amino acid sequence which differs from SEQ ID NO: 1 by no more than 2 (e.g., 0, 1 and 2) amino acid additions, deletions and/or substitutions;
(ii) a CDR2 comprising the amino acid sequence of SEQ ID NO: 2;
   a CDR2 comprising an amino acid sequence having at least 80%, 85%, 90%, 95% or 99% identity to SEQ ID NO: 2; or
   a CDR2 comprising an amino acid sequence which differs from SEQ ID NO: 2 by no more than 2 (e.g., 0, 1, and 2) amino acid additions, deletions and/or substitutions;
      and
(iii) a CDR3 comprising the amino acid sequence of SEQ ID NO: 3 or 12;
   a CDR3 comprising an amino acid sequence having at least 80%, 85%, 90%, 95% or 99% identity to SEQ ID NO: 3 or 12; or
   a CDR3 comprising an amino acid sequence which differs from SEQ ID NO: 3 or 12 by no more than 2 (e.g., 0, 1, and 2) amino acid additions, deletions and/or substitutions.

In some embodiments, the PD-L1 binding molecule comprises a heavy chain variable region, wherein the heavy chain variable region comprises:
(i) a CDR1 as shown in formula RTDSNIX₁GMH, wherein X₁ is H, F or N;
(ii) a CDR2 as shown in formula TIFIDX₂NTX₃, wherein X₂ is G, L or A, and X₃ is I or L; and
(iii) a CDR3 as shown in formula DVSGYGRX₄, wherein X₄ is A or Y.

In some embodiments, the PD-L1 binding molecule comprises a heavy chain variable region, wherein the heavy chain variable region comprises:
(i) a CDR1 comprising or consisting of SEQ ID NO: 1;
(ii) a CDR2 comprising or consisting of SEQ ID NO: 2; and
(iii) a CDR3 comprising or consisting of SEQ ID NO: 3.

In some embodiments, the PD-L1 binding molecule comprises a heavy chain variable region, wherein the heavy chain variable region comprises:
(i) a CDR1 comprising or consisting of SEQ ID NO: 4;
(ii) a CDR2 comprising or consisting of SEQ ID NO: 5; and
(iii) a CDR3 comprising or consisting of SEQ ID NO: 3.

In some embodiments, the PD-L1 binding molecule comprises a heavy chain variable region, wherein the heavy chain variable region comprises:
(i) a CDR1 comprising or consisting of SEQ ID NO: 6;
(ii) a CDR2 comprising or consisting of SEQ ID NO: 7; and
(iii) a CDR3 comprising or consisting of SEQ ID NO: 3.

In some embodiments, the PD-L1 binding molecule comprises a heavy chain variable region, wherein the heavy chain variable region comprises:
(i) a CDR1 comprising or consisting of SEQ ID NO: 1;
(ii) a CDR2 comprising or consisting of SEQ ID NO: 8; and
(iii) a CDR3 comprising or consisting of SEQ ID NO: 9.

In some embodiments, the PD-L1 binding molecule comprises a heavy chain variable region, wherein the heavy chain variable region comprises:
(i) a CDR1 comprising or consisting of SEQ ID NO: 10;
(ii) a CDR2 comprising or consisting of SEQ ID NO: 11; and
(iii) a CDR3 comprising or consisting of SEQ ID NO: 12.

In some embodiments, the PD-L1 binding molecule comprises a heavy chain variable region, wherein the heavy chain variable region comprises:
(i) a CDR1 comprising or consisting of SEQ ID NO: 13;
(ii) a CDR2 comprising or consisting of SEQ ID NO: 5; and
(iii) a CDR3 comprising or consisting of SEQ ID NO: 3.

In some embodiments, the PD-L1 binding molecule comprises a heavy chain variable region, wherein the heavy chain variable region comprises:
(i) a CDR1 comprising or consisting of SEQ ID NO: 14;
(ii) a CDR2 comprising or consisting of SEQ ID NO: 15; and
(iii) a CDR3 comprising or consisting of SEQ ID NO: 3.

In some embodiments, the PD-L1 binding molecule comprises a heavy chain variable region, wherein the heavy chain variable region comprises:
(i) a CDR1 comprising or consisting of SEQ ID NO: 16;
(ii) a CDR2 comprising or consisting of SEQ ID NO: 17; and
(iii) a CDR3 comprising or consisting of SEQ ID NO: 3.

In some embodiments, the PD-L1 binding molecule comprises a heavy chain variable region, wherein the heavy chain variable region comprises:
(i) a CDR1 comprising or consisting of SEQ ID NO: 18;
(ii) a CDR2 comprising or consisting of SEQ ID NO: 19; and
(iii) a CDR3 comprising or consisting of SEQ ID NO: 3.

In some embodiments, the PD-L1 binding molecule comprises a heavy chain variable region, wherein the heavy chain variable region comprises:
(i) a CDR1 comprising or consisting of SEQ ID NO: 20;
(ii) a CDR2 comprising or consisting of SEQ ID NO: 5; and
(iii) a CDR3 comprising or consisting of SEQ ID NO: 3.

In some aspects, the present invention provides an isolated PD-L1 binding molecule, which specifically binds to PD-L1 and comprises a heavy chain variable region, wherein the heavy chain variable region comprises:
(i) a CDR1 comprising or consisting of SEQ ID NO: 21;
(ii) a CDR2 comprising or consisting of SEQ ID NO: 22; and
(iii) a aCDR3 comprising or consisting of SEQ ID NO: 23.

In some aspects, the present invention provides an isolated PD-L1 binding molecule, which specifically binds to PD-L1 and comprises a heavy chain variable region, wherein the heavy chain variable region comprises:
(i) a CDR1 comprising or consisting of SEQ ID NO: 24;
(ii) a CDR2 comprising or consisting of SEQ ID NO: 25; and
(iii) a CDR3 comprising or consisting of SEQ ID NO: 3.

In some aspects, the present invention provides an isolated PD-L1 binding molecule, which specifically binds to PD-L1 and comprises a heavy chain variable region, wherein the heavy chain variable region comprises:
(i) a CDR1 comprising or consisting of SEQ ID NO: 26;
(ii) a CDR2 comprising or consisting of SEQ ID NO: 27; and
(iii) a CDR3 comprising or consisting of SEQ ID NO: 23.

In some aspects, the present invention provides an isolated PD-L1 binding molecule, which specifically binds to PD-L1 and comprises a heavy chain variable region, wherein the heavy chain variable region comprises:
(i) a CDR1 comprising or consisting of SEQ ID NO: 28;
(ii) a CDR2 comprising or consisting of SEQ ID NO: 29; and
(iii) a CDR3 comprising or consisting of SEQ ID NO: 23.

In some aspects, the present invention provides an isolated PD-L1 binding molecule, which specifically binds to PD-L1 and comprises a heavy chain variable region, wherein the heavy chain variable region comprises:
(i) a CDR1 comprising or consisting of SEQ ID NO: 30;
(ii) a CDR2 comprising or consisting of SEQ ID NO: 31; and
(iii) a CDR3 comprising or consisting of SEQ ID NO: 23.

In some aspects, the present invention provides an isolated PD-L1 binding molecule, which specifically binds to PD-L1 and comprises a heavy chain variable region, wherein the heavy chain variable region comprises 3 CDRs in a heavy chain variable region amino acid sequence of any one of SEQ ID NOs: 36-52.

In some embodiments, in the isolated PD-L1 binding molecule of the present invention, the heavy chain variable region further comprises an FR region comprising FR1, FR2, FR3 and FR4, and the FR1, FR2, FR3 and FR4 and CDR1, CDR2 and CDR3 are arranged alternately on the heavy chain variable region to form a structure of FR1-CDR1-FR2-CDR2-FR3-CDR3-FR4 from N-terminus to C-terminus.

In a preferred embodiment, in the isolated PD-L1 binding molecule of the present invention, the FR region comprises:
(a) an FR1 comprising the amino acid sequence of SEQ ID NO: 53;
   an FR1 comprising an amino acid sequence having at least 90%, 95% or 99% identity to SEQ ID NO: 53; or
   an FR1 comprising an amino acid sequence which differs from SEQ ID NO: 53 by no more than 2 (e.g., 0, 1 and 2) amino acid additions, deletions and/or substitutions;
(b) an FR2 comprising the amino acid sequence of SEQ ID NO: 54;
   an FR2 comprising an amino acid sequence having at least 90%, 95% or 99% identity to SEQ ID NO: 54; or
   an FR2 comprising an amino acid sequence which differs from SEQ ID NO: 54 by no more than 2 (e.g., 0, 1 and 2) amino acid additions, deletions and/or substitutions;
(c) an FR3 comprising the amino acid sequence of SEQ ID NO: 55;
   an FR3 comprising an amino acid sequence having at least 90%, 95% or 99% identity to SEQ ID NO: 55; or
   an FR3 comprising an amino acid sequence which differs from SEQ ID NO: 55 by no more than 2 (e.g., 0, 1 and 2) amino acid additions, deletions and/or substitutions;
      and
(d) an FR4 comprising the amino acid sequence as shown in SEQ ID NO: 56;
   an FR4 comprising an amino acid sequence having at least 90%, 95% or 99% identity to SEQ ID NO: 56; or
   an FR4 comprising an amino acid sequence which differs from SEQ ID NO: 56 by no more than 2 (e.g., 0, 1, and 2) amino acid additions, deletions and/or substitutions.

In a preferred embodiment, in the isolated PD-L1 binding molecule of the present invention, the FR region comprises:
(a) an FR1 comprising the amino acid sequence of SEQ ID NO: 57;
   an FR1 comprising an amino acid sequence having at least 90%, 95% or 99% identity to SEQ ID NO: 57; or
   an FR1 comprising an amino acid sequence which differs from SEQ ID NO: 57 by no more than 2 (e.g., 0, 1 and 2) amino acid additions, deletions and/or substitutions;
(b) an FR2 comprising the amino acid sequence as shown in SEQ ID NO: 58;
   an FR2 comprising an amino acid sequence having at least 90%, 95% or 99% identity to SEQ ID NO: 58; or
   an FR2 comprising an amino acid sequence which differs from SEQ ID NO: 58 by no more than 2 (e.g., 0, 1 and 2) amino acid additions, deletions and/or substitutions;
(c) an FR3 comprising the amino acid sequence of SEQ ID NO: 59;
   an FR3 comprising an amino acid sequence having at least 90%, 95% or 99% identity to SEQ ID NO: 59; or
   an FR3 comprising an amino acid sequence which differs from SEQ ID NO: 59 by no more than 2 (e.g., 0, 1 and 2) amino acid additions, deletions and/or substitutions of amino acids;
      and
(d) an FR4 comprising the amino acid sequence of SEQ ID NO: 60;
   an FR4 comprising an amino acid sequence having at least 90%, 95% or 99% identity to SEQ ID NO: 60; or
   an FR4 comprising an amino acid sequence which differs from SEQ ID NO: 60 by no more than 2 (e.g., 0, 1, and 2) amino acid additions, deletions and/or substitutions.

In a preferred embodiment, in the isolated PD-L1 binding molecule of the present invention, the FR region comprises:
(a) an FR1 comprising the amino acid sequence of SEQ ID NO: 61;
   an FR1 comprising an amino acid sequence having at least 90%, 95% or 99% identity to SEQ ID NO: 61; or
   an FR1 comprising an amino acid sequence which differs from SEQ ID NO: 61 by no more than 2 (e.g., 0, 1 and 2) amino acid additions, deletions and/or substitutions;
(b) an FR2 comprising the amino acid sequenceof SEQ ID NO: 58;
   an FR2 comprising an amino acid sequence having at least 90%, 95% or 99% identity to SEQ ID NO: 58; or
   an FR2 comprising an amino acid sequence which differs from SEQ ID NO: 58 by no more than 2 (e.g., 0, 1 and 2) amino acid additions, deletions and/or substitutions;
(c) an FR3 comprising the amino acid sequence of SEQ ID NO: 59;
   an FR3 comprising an amino acid sequence having at least 90%, 95% or 99% identity to SEQ ID NO: 59; or
   an FR3 comprising an amino acid sequence which differs from SEQ ID NO: 59 by no more than 2 (e.g., 0, 1 and 2) amino acid additions, deletions and/or substitutions;
      and
(d) an FR4 comprising the amino acid sequence of SEQ ID NO: 60;
   an FR4 comprising an amino acid sequence having at least 90%, 95% or 99% identity to SEQ ID NO: 60; or
   an FR4 comprising an amino acid sequence which differs from SEQ ID NO: 60 by no more than 2 (e.g., 0, 1, and 2) amino acid additions, deletions and/or substitutions of amino acids.

In some embodiments, in the isolated PD-L1 binding molecule of the present invention, the heavy chain variable region comprises or consists of an amino acid sequence of any one of SEQ ID NOs: 36-52.

In some embodiments, in the isolated PD-L1 binding molecule of the present invention, the heavy chain variable region comprises an amino acid sequence having at least 80%, 85%, 90%, 95% or 99% identity to an amino acid sequence as shown in any one of SEQ ID NOs: 36-52 and retaining the ability to specifically bind to PD-L1.

In some embodiments, in the isolated PD-L1 binding molecule of the present invention, the heavy chain variable region comprises an amino acid sequence having one or more amino acid additions, deletions and/or substitutions (e.g., conservative substitutions) compared to an amino acid sequence of any one of SEQ ID NOs: 36-52 and retaining the ability to specifically bind to PD-L1.

In a preferred embodiment, the one or more additions, deletions and/or substitutions (e.g., conservative substitutions) of amino acids are no more than five, preferably no more than three.

In some aspects, the isolated PD-L1 binding molecule of the present invention is a camelized antibody, a humanized antibody, an affinity-matured antibody or a druggability-modified molecule.

In some aspects, the isolated PD-L1 binding molecule of the present invention is a single-domain antibody or an antigen-binding fragment thereof. Therefore, the present invention also provides an isolated PD-L1 single-domain antibody in a nanobody format.

In some aspects, the heavy chain variable region of the isolated PD-L1 binding molecule of the present invention can be fused to an additional molecule, and the additional molecule is selected from an Fc domain of an immunoglobulin (e.g., IgG), an antibody, an antigen-binding fragment of the antibody, an antibody-drug conjugate, an antibody-like molecule, an antigen-binding fragment of the antibody-like molecule or a fluorescent protein.

In some embodiments, the PD-L1 binding molecule is fused to an Fc domain of human IgG (e.g., human IgG1 or human IgG4).

In some aspects, the present invention provides an isolated nucleic acid molecule, comprising a nucleotide sequence encoding the isolated PD-L1 binding molecule of the present invention.

In some aspects, the present invention provides a vector, wherein the vector comprises a nucleic acid molecule comprising a nucleotide sequence encoding the isolated PD-L1 binding molecule of the present invention.

In some aspects, the present invention provides a host cell, wherein the host cell comprises a nucleic acid molecule comprising a nucleotide sequence encoding the isolated PD-L1 binding molecule of the present invention or comprises the vector of the present invention.

In some aspects, the present invention provides a pharmaceutical composition, wherein the pharmaceutical composition comprises the PD-L1 binding molecule of the present invention and a pharmaceutically acceptable carrier.

In some embodiments, the pharmaceutical composition comprises the PD-L1 single-domain antibody or the antigen-binding fragment thereof of the present invention and a pharmaceutically acceptable carrier.

In some aspects, the present invention provides a method for preparing the PD-L1 binding molecule of the present invention, wherein the method comprises the following steps:
(1) expressing the nucleotide sequence encoding the isolated PD-L1 binding molecule of the present invention in a suitable host cell to produce the PD-L1 binding molecule; and
(2) isolating the PD-L1 binding molecule from the host cell.

In some aspects, the present invention relates to a method for modulating an immune response in a subject, comprising administering the PD-L1 binding molecule as disclosed herein to the subject, such that the immune response in the subject is modulated.

In some embodiments, the subject is a human or a mammal suffering from a disease associated with PD-L1. Specifically, the subject may suffer from diseases including but not limited to the following: renal cell carcinoma, non-small cell lung cancer, bladder cancer, urothelial carcinoma, microsatellite unstable solid tumor, etc.

In some aspects, the present invention relates to a method for treating or preventing a disease associated with PD-L1, comprising administering an effective amount of the PD-L1 binding molecule as disclosed herein, or administering the PD-L1 single-domain antibody or the antigen-binding fragment thereof as disclosed herein, or administering an effective amount of a pharmaceutical composition comprising the PD-L1 binding molecule as disclosed herein, or administering an effective amount of a pharmaceutical composition comprising the PD-L1 single-domain antibody or the antigen-binding fragment thereof as disclosed herein, to a patient suffering from the disease associated with PD-L1 or a subject with a propensity to suffer from the disease associated with PD-L1.

In some aspects, the present invention relates to a method for treating any disease or condition that can be improved, slowed, inhibited or prevented by eliminating, inhibiting or reducing PD-L1 activity.

In some other aspects, the method of the present invention further relates to a method for treating or preventing tumors by combination therapy, the method comprising administering an effective amount of the PD-L1 binding molecule, the PD-L1 single-domain antibody or the antigen-binding fragment thereof described herein and one or more other drugs to a subject.

In some embodiments, the method disclosed herein further comprises administering in combination an effective amount of a second drug to a subject, wherein the PD-L1 binding molecule or the PD-L1 single-domain antibody or the antigen-binding fragment thereof disclosed herein is a first drug. In one embodiment, the second drug is a chemotherapeutic agent, a radiotherapeutic agent, or a biomacromolecular drug for the treatment of a disease associated with PD-L1. In one embodiment, the biomacromolecular drug is, for example, various monoclonal antibody drugs (e.g., rituximab, cetuximab and trastuzumab) that attack tumor cells by T cell recognition. As used herein, the expression "a second drug" does not mean that it refers to the only drug other than the first drug. Thus, the second drug need not be one drug, but may constitute or contain more than one such drug.

In some embodiments, the subject, patient or individual is a mammal, such as a mouse or a human, preferably a human.

In some aspects, the present invention relates to the use of the PD-L1 binding molecule as disclosed herein in the preparation of a drug for treating or preventing a disease associated with PD-L1.

In some aspects, the present invention relates to the use of the PD-L1 single-domain antibody or the antigen-binding fragment thereof as disclosed herein in the preparation of a drug for treating or preventing a disease associated with PD-L1.

In some embodiments, the disease associated with PD-L1 is selected from, but is not limited to renal cell carcinoma, non-small cell lung cancer, bladder cancer, urothelial carcinoma, microsatellite unstable solid tumor, etc.

In some aspects, the present invention relates to a kit or a device and a related method of using the PD-L1 binding molecule, the PD-L1 single-domain antibody or the antigen-binding fragment thereof as disclosed herein, and the pharmaceutical composition as disclosed herein, which can be used for treating a disease associated with PD-L1, such as cancers. To this end, the present invention preferably provides an article that can be used for treating such conditions, which comprises a container comprising the PD-L1 binding molecule, the PD-L1 single-domain antibody or the antigen-binding fragment thereof as disclosed herein, and an explanatory material of using the PD-L1 single-domain antibody or the antigen-binding fragment thereof as disclosed herein to treat, improve or prevent a disease associated with PD-L1 or progression or relapse thereof.

The present invention also encompasses any combinations of any embodiments described herein. Any embodiments or any combinations thereof described herein are applicable to any and all PD-L1 binding molecules, PD-L1 single-domain antibodies or antigen-binding fragments thereof, methods and uses thereof of the present invention described herein.

### Brief Description of the Drawings

Figure 1 shows the results of screening for binding affinity of VHH antibody lysate samples that bind to PD-L1.
Figure 2 shows the screening results of blocking assay of anti-PD-Ll candidate antibody molecules.
Figure 3 shows the verification results of cell binding assay of candidate antibody molecules: (A) isotype control, (B) NB22D-21, (C) NB22gb-10, and (D) positive control KN035.
Figure 4 shows the verification results of specific binding reaction assay of the NB22D-21 molecule.
Figure 5 shows the verification results of mixed lymphocyte reaction assay of the NB22D-21 molecule.
Figure 6 shows the verification results of binding assay of the NB22D-21 molecule and the humanized modified derivative molecules thereof.
Figure 7 shows the results of human-mouse cross reaction assay of the NB22D-21 molecule and the humanized modified derivative molecules thereof.
Figure 8 shows the results of the peak plots in flow cytometry of human-mouse cross reaction assay of the humanized modified derivative molecule NB22D-21-huVH1: (A) control molecule KN035, and (B) NB22D-21-huVH1.
Figure 9 shows the results of blocking activity on binding of the NB22D-21 molecule and the humanized modified molecules thereof.
Figure 10 shows the results of affinity assay of the affinity-matured molecules on PD-L1-CHO.
Figure 11 shows the binding of PD-1 to PD-L1-CHO cells blocked by the affinity-matured molecules.
Figure 12 shows the affinity assay of the second affinity-matured molecules binding to human PD-L1-CHO cells.
Figure 13 shows the blocking activity assay of the second affinity-matured molecules in PD-1 binding to human PD-L1-CHO cells.
Figure 14 shows the binding assay of the second affinity-matured molecules to mouse PD-L1 cells.
Figure 15 shows the blocking activity of druggability-modified molecules in PD-L1-CHO, wherein the isotype control is human IgG1.
Figure 16 shows the effect of druggability-modified molecules on the secretion of IFN-γ (A) and IL-2 (B) in the mixed lymphocyte reaction.

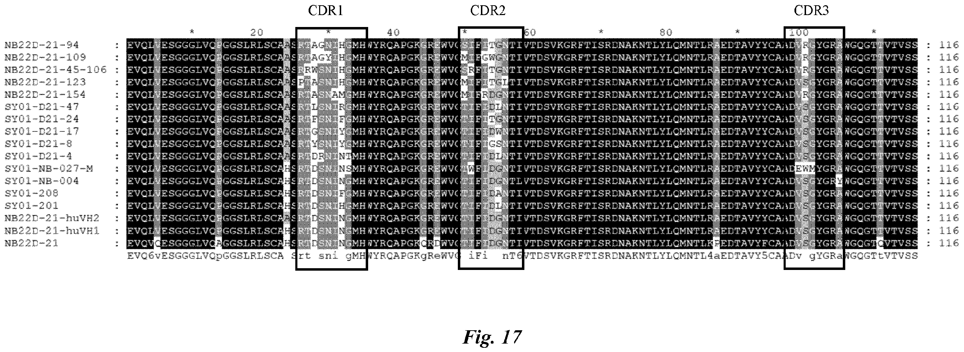

Figure 17 shows the sequence alignment of candidate antibody molecules, wherein the CDR sequences are marked with boxes.

### Overview of the Sequence Listing

The present application is accompanied by a Sequence Listing containing numerous nucleotide and amino acid sequences. Tables A, B and C below provide an overview of the sequences included.

**Table A. Heavy chain variable region CDR sequences of antibodies**

| **Description** | **Clone name** | **SEQ ID NO:** | **CDR1** | **SEQ ID NO:** | **CDR2** | **SEQ ID NO:** | **CDR3** |
|---|---|---|---|---|---|---|---|
| Positive control | KN035 | 32 | GFTFSRRCKA | 33 | KILTTSGSTY | 34 | DSFEDPTCTLVTSSGAFQY |
| Alpaca screening | NB22D-21 | 1 | RTDSNINGMH | 2 | TIFIDGNTI | 3 | DVSGYGRA |
| Humanization | NB22D-21-huVH2 | 1 | RTDSNINGMH | 2 | TIFIDGNTI | 3 | DVSGYGRA |
| Humanization | NB22D-21-huVH1 | 1 | RTDSNINGMH | 2 | TIFIDGNTI | 3 | DVSGYGRA |
| Affinity maturation | SY01-201 | 4 | RTDSNIHGMH | 5 | TIFIDLNTI | 3 | DVSGYGRA |
| Affinity maturation | SY01-208 | 6 | RTDSNIFGMH | 7 | TIFIDANTI | 3 | DVSGYGRA |
| Affinity maturation | SY01-NB-004 | 1 | RTDSNINGMH | 8 | TIFIDGNTL | 9 | DVSGYGRY |
| Affinity maturation | SY01-NB-027-M | 10 | RTDSNINSMH | 11 | TWFIDGNTI | 12 | DEWMYGRA |
| Druggability modification | NB22D-21-4 | 13 | RTDRNINTMH | 5 | TIFIDLNTI | 3 | DVSGYGRA |
| Druggability modification | NB22D-21-8 | 14 | RTYSNIYGMH | 15 | TIFIGSNTI | 3 | DVSGYGRA |
| Druggability modification | NB22D-21-17 | 16 | RTGSNIYGMH | 17 | TIFIDWNTI | 3 | DVSGYGRA |
| Druggability modification | NB22D-21-24 | 18 | RTFSNIFGMH | 19 | TIFITGNTI | 3 | DVSGYGRA |
| Druggability modification | NB22D-21-47 | 20 | RTLSNIRGMH | 5 | TIFIDLNTI | 3 | DVSGYGRA |
| Affinity maturation | NB22D-21-45-106 | 21 | RRWSNIHGMH | 22 | SRFITGNTI | 23 | DVRGYGRA |
| Affinity maturation | NB22D-21-123 | 24 | PTASNIHGMH | 25 | MIFITGL TI | 3 | DVSGYGRA |
| Affinity maturation | NB22D-21-154 | 26 | RTASNAMGMH | 27 | MIFRDGNTI | 23 | DVRGYGRA |
| Affinity maturation | NB22D-21-94 | 28 | RTAGNIHGMH | 29 | SIFITGNTI | 23 | DVRGYGRA |
| Affinity maturation | NB22D-21-109 | 30 | RTAGYIHGMH | 31 | MIFGWGNTI | 23 | DVRGYGRA |

**Table B. VH sequences of antibodies, with the CDRs marked with boxes**

| **Description** | **Clone** | **SEQ ID NO:** | **VH region** |
|---|---|---|---|
| Positive control | KN035 | 35 | QVQLVESGGGLVQPGGSLRLSCAASGFTFSRRCKAWFRQAPGKERERVAKILTTSGSTYLADSVK GRFTISRDNSKNTVYLQMNSLRAEDTAVYYCAADSFEDPTCTLVTSSGAFQYWGQGTLVTVSS |
| Alpaca screening | NB22D-21 | 36 | EVQVQESGGGLVQAGGSLRLSCAHSRTDSNINGMHWYRQAPGKQRDWVGTIFIDGNTIVTDSVK GRFTISRDNAKNTLYLQMNTLKPEDTAVYFCAADVSGYGRAWGQGTQVTVSS |
| Humanization | NB22D-21-huVH2 | 37 | EVQLVESGGGLVQPGGSLRLSCAASRTDSNINGMHWYRQAPGKGREWVGTIFIDGNTIVTDSVKG RFTISRDNAKNTLYLQMNTLRAEDTAVYYCAADVSGYGRAWGQGTTVTVSS |
| Humanization | NB22D-21-nuvH1 | 38 | EVQLVESGGGLVQPGGSLRLSCAHSRTDSNINGMHWYRQAPGKGREWVGTIFIDGNTIVTDSVKG RFTISRDNAKNTLYLQMNTLRAEDTAVYYCAADVSGYGRAWGQGTTVTVSS |
| Affinity maturation | SY01-201 | 39 | EVQLVESGGGLVQPGGSLRLSCAHSRTDSNIHGMHWYRQAPGKGREWVGTIFIDLNTIVTDSVKG RFTISRDNAKNTLYLQMNTLRAEDTAVYYCAADVSGYGRAWGQGTTVTVSS |
| Affinity maturation | SY01-208 | 40 | EVQLVESGGGLVQPGGSLRLSCAHSRTDSNIFGMHWYRQAPGKGREWVGTIFIDANTIVTDSVKGR FTISRDNAKNTLYLQMNTLRAEDTAVYYCAADVSGYGRAWGQGTTVTVSS |
| Affinity maturation | SY01-NB-004 | 41 | EVQLVESGGGLVQPGGSLRLSCAHSRTDSNINGMHWYRQAPGKGREWVGTIFIDGNTLVTDSVKG RFTISRDNAKNTLYLQMNTLRAEDTAVYYCAADVSGYGRYWGQGTTVTVSS |
| Affinity maturation | SY01-NB-027-M | 42 | EVQLVESGGGLVQPGGSLRLSCAHSRTDSNINSMHWYRQAPGKGREWVGTWFIDGNTIVTDSVKG RFTISRDNAKNTLYLQMNTLRAEDTAVYYCAADEWMYGRAWGQGTTVTVSS |
| Druggability modification | SY01-D21-4 | 43 | EVQLVESGGGLVQPGGSLRLSCAASRTDRNINTMHWYRQAPGKGREWVGTIFIDLNTIVTDSVKG RFTISRDNAKNTLYLQMNTLRAEDTAVYYCAADVSGYGRAWGQGTTVTVSS |
| Druggability modification | SY01-D21-8 | 44 | EVQLVESGGGLVQPGGSLRLSCAASRTYSNIYGMHWYRQAPGKGREWVGTIFIGSNTIVTDSVKGR FTISRDNAKNTLYLQMNTLRAEDTAVYYCAADVSGYGRAWGQGTTVTVSS |
| Druggability modification | SY01-D21-17 | 45 | EVQLVESGGGLVQPGGSLRLSCAASRTGSNIYGMHWYRQAPGKGREWVGTIFIDWNTIVTDSVKG RFTISRDNAKNTLYLQMNTLRAEDTAVYYCAADVSGYGRAWGQGTTVTVSS |
| Druggability modification | SY01-D21-24 | 46 | EVQLVESGGGLVQPGGSLRLSCAASRTFSNIFGMHWYRQAPGKGREWVGTIFITGNTIVTDSVKGR FTISRDNAKNTLYLQMNTLRAEDTAVYYCAADVSGYGRAWGQGTTVTVSS |
| Druggability modification | SY01-D21-47 | 47 | EVQLVESGGGLVQPGGSLRLSCAASRTLSNIRGMHWYRQAPGKGREWVGTIFIDLNTIVTDSVKGR FTISRDNAKNTLYLQMNTLRAEDTAVYYCAADVSGYGRAWGQGTTVTVSS |
| Affinity maturation | NB22D-21-45-106 | 48 | EVQLVESGGGLVQPGGSLRLSCAASRRWSNIHGMHWYRQAPGKGREWVGSRFITGNTIVTDSVKG RFTISRDNAKNTLYLQMNTLRAEDTAVYYCAADVRGYGRAWGQGTTVTVSS |
| Affinity maturation | NB22D-21-123 | 49 | EVQLVESGGGLVQPGGSLRLSCAASPTASNIHGMHWYRQAPGKGREWVGMIFITGLTIVTDSVKG RFTISRDNAKNTLYLQMNTLRAEDTAVYYCAADVSGYGRAWGQGTTVTVSS |
| Affinity maturation | NB22D-21-154 | 50 | EVQLVESGGGLVQPGGSLRLSCAASRTASNAMGMHWYRQAPGKGREWVGMIFRDGNTIVTDSVK GRFTISRDNAKNTLYLQMNTLRAEDTAVYYCAADVRGYGRAWGQGTTVTVSS |
| Affinity maturation | NB22D-21-94 | 51 | EVQLVESGGGLVQPGGSLRLSCAASRTAGNIHGMHWYRQAPGKGREWVGSIFITGNTIVTDSVKG RFTISRDNAKNTLYLQMNTLRAEDTAVYYCAADVRGYGRAWGQGTTVTVSS |
| Affinity maturation | NB22D-21-109 | 52 | EVQLVESGGGLVQPGGSLRLSCAASRTAGYIHGMHWYRQAPGKGREWVGMIFGWGNTIVTDSV KGRFTISRDNAKNTLYLQMNTLRAEDTAVYYCAADVRGYGRAWGQGTTVTVSS |

| | | | |
|---|---|---|---|
| **Notes: the CDR1-3 sequences are marked with boxes.** | | | |

**Table C. FR sequences of antibodies**

| **Description** | **Clone name** | **SEQID NO:** | **FR1** | **SEQID NO:** | **FR2** | **SEQ IDNO:** | **FR3** | **SEQID NO:** | **FR4** |
|---|---|---|---|---|---|---|---|---|---|
| Positivecontrol | KN035 | 62 | QVQLVESGGGLV QPGGSLRLSCAAS | 63 | WFRQAPGK ERERVA | 64 | LADSVKGRFTISRDNSKN TVYLQMNSLRAEDTAVY YCAA | 65 | WGQGTL VTVSS |
| Alpacascreening | NB22D-21 | 53 | EVQVQESGGGLV QAGGSLRLSCAHS | 54 | WYRQAPGK QRDWVG | 55 | VTDSVKGRFTISRDNAKN TLYLQMNTLKPEDTAVYF CAA | 56 | WGQGTQ VTVSS |
| Humanization | NB22D-21-huVH2 | 57 | | 58 | WYRQAPGK GREWVG | 59 | VTDSVKGRFTISRDNAKN TLYLQMNTLRAEDTAVY YCAA | 60 | WGQGTT VTVSS |
| Humanization | NB22D-21-huVH1 | 61 | EVQLVESGGGLV QPGGSLRLSCAHS | 58 | WYRQAPGK GREWVG | 59 | VTDSVKGRFTISRDNAKN TLYLQMNTLRAEDTAVY YCAA | 60 | WGQGTT VTVSS |
| Affinity maturation | SY01-201 | 61 | EVQLVESGGGLV QPGGSLRLSCAHS | 58 | WYRQAPGK GREWVG | 59 | VTDSVKGRFTISRDNAKN TLYLQMNTLRAEDTAVY YCAA | 60 | WGQGTT VTVSS |
| Affinity maturation | SY01-208 | 61 | EVQLVESGGGLV QPGGSLRLSCAHS | 58 | WYRQAPGK GREWVG | 59 | VTDSVKGRFTISRDNAKN TLYLQMNTLRAEDTAVY YCAA | 60 | WGQGTT VTVSS |
| Affinity maturation | SY01-NB-004 | 61 | EVQLVESGGGLV QPGGSLRLSCAHS | 58 | WYRQAPGK GREWVG | 59 | VTDSVKGRFTISRDNAKN TLYLQMNTLRAEDTAVY YCAA | 60 | WGQGTT VTVSS |
| Affinity maturation | SY01-NB-027-M | 61 | EVQLVESGGGLV QPGGSLRLSCAHS | 58 | WYRQAPGK GREWVG | 59 | VTDSVKGRFTISRDNAKN TLYLQMNTLRAEDTAVY YCAA | 60 | WGQGTT VTVSS |
| Druggability modification | NB22D-21-4 | 57 | | 58 | WYRQAPGK GREWVG | 59 | VTDSVKGRFTISRDNAKN TLYLQMNTLRAEDTAVY YCAA | 60 | WGQGTT VTVSS |
| Druggability modification | NB22D-21-8 | 57 | | 58 | WYRQAPGK GREWVG | 59 | VTDSVKGRFTISRDNAKN TLYLQMNTLRAEDTAVY YCAA | 60 | WGQGTT VTVSS |
| Druggability modification | NB22D-21-17 | 57 | | 58 | WYRQAPGK GREWVG | 59 | VTDSVKGRFTISRDNAKN TLYLQMNTLRAEDTAVY YCAA | 60 | WGQGTT VTVSS |
| Druggability modification | NB22D-21-24 | 57 | | 58 | WYRQAPGK GREWVG | 59 | VTDSVKGRFTISRDNAKN TLYLQMNTLRAEDTAVY YCAA | 60 | WGQGTT VTVSS |
| Druggability modification | NB22D-21-47 | 57 | | 58 | WYRQAPGK GREWVG | 59 | VTDSVKGRFTISRDNAKN TLYLQMNTLRAEDTAVY YCAA | 60 | WGQGTT VTVSS |
| Affinity maturation | NB22D-21-45-106 | 57 | | 58 | WYRQAPGK GREWVG | 59 | VTDSVKGRFTISRDNAKN TLYLQMNTLRAEDTAVY YCAA | 60 | WGQGTT VTVSS |
| Affinity maturation | NB22D-21-123 | 57 | | 58 | WYRQAPGK GREWVG | 59 | VTDSVKGRFTISRDNAKN TLYLQMNTLRAEDTAVY YCAA | 60 | WGQGTT VTVSS |
| Affinity maturation | NB22D-21-154 | 57 | | 58 | WYRQAPGK GREWVG | 59 | VTDSVKGRFTISRDNAKN TLYLQMNTLRAEDTAVY YCAA | 60 | WGQGTT VTVSS |
| Affinity maturation | NB22D-21-94 | 57 | | 58 | WYRQAPGK GREWVG | 59 | VTDSVKGRFTISRDNAKN TLYLQMNTLRAEDTAVY YCAA | 60 | WGQGTT VTVSS |
| Affinity maturation | NB22D-21-109 | 57 | | 58 | WYRQAPGK GREWVG | 59 | VTDSVKGRFTISRDNAKN TLYLQMNTLRAEDTAVY YCAA | 60 | WGQGTT VTVSS |

### Detailed Description of Embodiments

It is to be understood by a person skilled in the art that the present invention is not limited to the specific methodology, embodiments and reagents described herein, because these are exemplary illustrations. It is also to be understood that the terminology used herein is merely for description of specific embodiments, and is not intended to limit the scope of the present invention which is defined only by the appended claims.

Unless defined otherwise, all technical and scientific terms used herein have the same meanings as commonly understood by one of ordinary skill in the art to which the present invention pertains.

In addition, unless the context requires otherwise, terms in the singular form shall include the plural form, and terms in the plural form shall include the singular form. More specifically, as used in the present specification and in the appended claims, the singular forms "a/an" and "the" include plural referents unless the context clearly dictates otherwise. Therefore, for example, reference to "an antibody" includes multiple antibodies.

### I. Definitions

To better understand the present invention, definitions and explanations of related terms are provided below.

The term "about" when used in connection with a numerical value is meant to encompass numerical values within a range having a lower limit that is 5% smaller than the specified numerical value and an upper limit that is 5% larger than the specified numerical value.

The term "antibody" herein is used in the broadest sense and encompasses various antibody structures, including but not limited to monoclonal antibodies, polyclonal antibodies, multispecific antibodies (e.g., bispecific antibodies) and antibody fragments so long as they exhibit the desired antigen-binding activity. A complete antibody generally will comprise at least two full-length heavy chains and two full-length light chains, but in some instances may include fewer chains, for example, naturally-occurring antibodies in camelids may comprise only heavy chains.

As used herein, the term "antigen-binding moiety" refers to a moiety that specifically binds to a target antigen. The term includes antibodies and other natural molecules (e.g., receptors and ligands) or synthetic molecules (e.g. DARPins) capable of specifically binding to a target antigen. In one preferred embodiment, the antigen-binding moiety of the antibody of the present invention is an antibody fragment.

The terms "full-length antibody", "intact antibody", and "complete antibody" are used herein interchangeably to refer to an antibody having a structure substantially similar to that of a natural antibody or having heavy chains that contain an Fc region.

As used herein, the term "monoclonal antibody" or "monoclonal antibody composition" refers to a preparation of an antibody molecule having a single amino acid composition, not to the method by which it is produced. Monoclonal antibodies or antigen-binding fragments thereof can be produced, for example, by hybridoma techniques, recombinant techniques, phage display techniques, synthetic techniques such as CDR grafting, or combinations of such or other techniques known in the art.

As used herein, the term "PD-1" refers to a programmed cell death protein, which belongs to an immunoglobulin superfamily and functions as a co-inhibitory receptor to negatively modulate the immune system. PD-1 is a member of the CD28/CTLA-4 family, and has two known ligands including PD-L1 and PD-L2. Alternative names or synonyms for PD-1 include PDCD1, PD1, CD279, SLEB2, etc. A representative amino acid sequence of human PD-1 is disclosed under the NCBI accession number: NP_005009.2, and a representative nucleic acid sequence encoding the human PD-1 is shown under the NCBI accession number: NM 005018.3.

As used herein, the term "PD-L1" refers to programmed cell death ligand 1 (PD-L1, see, for example, Freeman et al. (2000) J. Exp. Med. 192: 1027). Alternative names or synonyms for PD-L1 include PDCD1L1, PDL1, B7H1, CD274, B7-H, etc. A representative amino acid sequence of human PD-L1 is disclosed under the NCBI accession number: NP_054862.1, and a representative nucleic acid sequence encoding the human PD-L1 is shown under the NCBI accession number: NM 014143.4. PD-L1 is expressed in placenta, spleen, lymph nodes, thymus, heart and fetal liver, and is also found in many tumor or cancer cells. PD-L1 binds to its receptor PD-1 or B7-1 which is expressed on activated T cells, B cells and myeloid cells. The binding of PD-L1 to its receptor induces signal transduction to inhibit TCR-mediated activation of cytokine production and T cell proliferation. Accordingly, PD-L1 plays a major role in suppressing the immune system during particular events such as pregnancy, autoimmune diseases and tissue allografts, and is considered to allow tumor or cancer cells to bypass the immune checkpoints and evade the immune response.

As used herein, the terms "binding" and "specific binding" refer to the binding of an antibody or an antigen-binding moiety to an epitope in an *in vitro* assay, preferably in a bio-optical interferometry (ForteBio) with a purified wild-type antigen. In certain embodiments, an antibody or an antigen-binding moiety is said to specifically bind an antigen when it preferentially recognizes its target antigen in a complex mixture of proteins and/or macromolecules.

Antibodies are divided into the following "classes": IgA, IgD, IgE, IgG and IgM, depending on the amino acid sequence of the heavy chain constant region thereof, and several of these classes may be further divided into subclasses, such as IgG1, IgG2, IgG3, IgG4, IgA1 and IgA2. The heavy chain constant regions corresponding to different classes of antibodies are called [image] δ, ε, γ and µ, respectively. The light chain constant regions (CLs) that can be found in all five antibody classes are called κ and λ. Within full-length light and heavy chains, the variable and constant regions are typically linked by the "J" region of about 12 or more amino acids, and the heavy chain also includes the "D" region of about 10 or more amino acids. See, for example, Fundamental Immunology, Ch.7 (Paul, W. ed., 2nd edition, Raven Press, N.Y. (1989)) (which is incorporated herein by reference in its entirety for all purposes). The variable regions of each light/heavy chain pair typically form antigen-binding sites.

The term "variable region" or "variable domain" refers to an antibody heavy or light chain domain that is involved in the binding of an antibody to an antigen. The heavy and light chain variable domains of a natural antibody generally have similar structures, with each domain comprising four conserved framework regions (FRs) and three complementarity determining regions (CDRs) (see, for example, Kindt et al., Kuby Immunology, 6th ed., W.H. Freeman and Co., page 91 (2007)). A single VH or VL domain may be sufficient to confer antigen-binding specificity. Furthermore, an antibody that binds to a particular antigen may be isolated using a VH or VL domain from the antibody that binds to the antigen to screen a library of complementary VL or VH domains, respectively. See, for example, Portolano, et al., J. Immunol. 150: 880-887 (1993); Clarkson, et al., Nature 352: 624-628 (1991). The FRs and CDRs in the present disclosure are divided according to the AbM standard, but it must be pointed out that the CDRs in the present disclosure are not limited to the division method of AbM, and can further be divided according to any other conventional division methods in the art, such as Chothia, Martin, Kabat, AHo and IMGT.

The variable regions typically exhibit the same general structure as the relatively conserved framework regions (FRs) linked by three hypervariable regions which also called complementarity determining regions or CDRs. Generally, the CDRs from both chains of each pair are aligned by the framework regions, which CDRs allow the antibody to bind to a specific epitope. Two light and heavy chain variable regions typically comprise, from N-terminus to C-terminus, the domains FR1, CDR1, FR2, CDR2, FR3, CDR3 and FR4.

An "antibody fragment" refers to a molecule other than a complete antibody that comprises a portion of the complete antibody, which portion binds to the antigen to which the complete antibody binds.

"Affinity" refers to the strength of the sum of all non-covalent interactions between a single binding site of a molecule (e.g., an antibody) and a binding partner thereof (e.g., an antigen). Unless otherwise specified, "binding affinity" when used herein refers to an intrinsic binding affinity that reflects 1:1 interaction between members of a binding pair (e.g., an antibody and an antigen). The affinity of molecule X for the partner thereof, Y, can generally be represented by the dissociation constant (Kd). Affinity can be measured by common methods known in the art, including those known in the art and described herein.

As used herein, the term "EC₅₀", also referred to as "half maximal effective concentration", refers to the concentration of a drug, antibody or toxic agent which induces a response at 50% between the baseline and the maximum after a specific exposure time. In the context of the present application, the unit of EC₅₀ is "nM".

A "human antibody" refers to an antibody having an amino acid sequence corresponding to the amino acid sequence of an antibody produced by human or human cells or derived from a non-human source using human antibody repertoire or other human antibody coding sequences. This definition of a human antibody explicitly excludes a humanized antibody comprising non-human antigen-binding residues.

A "human consensus framework" refers to a framework representing the most frequently occurring amino acid residues in a selection of human immunoglobulin VL or VH framework sequences. Generally, the selection of human immunoglobulin VL or VH sequences is a selection from a subtype of variable domain sequences. Generally, the subtype of the sequences is the subtype as described in Kabat et al., Sequences of Proteins of Immunological Interest, 5th edition, NIH Publication 91-3242, Bethesda MD (1991), vol. 1-3. In one embodiment, for VL, the subtype is the subtype κ I as described in Kabat et al. (supra). In one embodiment, for VH, the subtype is the subtype III as described in Kabat et al. (supra).

A "humanized" antibody refers to a chimeric antibody comprising amino acid residues from non-human HVRs and amino acid residues from human FRs. In some embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody (e.g., a non-human antibody) refers to an antibody that has undergone humanization.

The term "conservative substitution" refers to substitution of an amino acid by another amino acid within the same class, for example, substitution of an acidic amino acid by another acidic amino acid, substitution of a basic amino acid by another basic amino acid, or substitution of a neutral amino acid by another neutral amino acid. Exemplary substitutions are shown in Table D below:

**Table D. Exemplary substitutions**

| **Original residue** | | **Exemplary substitution** | **Conservative substitution** |
|---|---|---|---|
| **Ala (A)** | **Val; Leu; Ile** | | **Val** |
| **Arg (R)** | **Lys; Gln; Asn** | | **Lys** |
| **Asn (N)** | **Gln; His; Asp, Lys; Arg** | | **Gln** |
| **Asp (D)** | **Glu; Asn** | | **Glu** |
| **Cys (C)** | **Ser; Ala** | | **Ser** |
| **Gln (Q)** | **Asn; Glu** | | **Asn** |
| **Glu (E)** | **Asp; Gln** | | **Asp** |
| **Gly (G)** | **Ala** | | **Ala** |
| **His (H)** | **Asn; Gln; Lys; Arg** | | **Arg** |
| **Ile (I)** | **Leu; Val; Met; Ala; Phe; Norleucine** | | **Leu** |
| **Leu (L)** | **Norleucine; Ile; Val; Met; Ala; Phe** | | **Ile** |
| **Lys (K)** | **Arg; Gln; Asn** | | **Arg** |
| **Met (M)** | **Leu; Phe; Ile** | | **Leu** |
| **Phe (F)** | **Trp; Leu; Val; Ile; Ala; Tyr** | | **Tyr** |
| **Pro (P)** | **Ala** | | **Ala** |
| **Ser (S)** | **Thr** | | **Thr** |
| **Thr (T)** | **Val; Ser** | | **Ser** |
| **Trp (W)** | **Tyr; Phe** | | **Tyr** |
| **Tyr (Y)** | **Trp; Phe; Thr; Ser** | | **Phe** |
| **Val (V)** | **Ile; Leu; Met; Phe; Ala; Norleucine** | | **Leu** |

Amino acids can be grouped according to the properties of common side chains:
(1) hydrophobic: Norleucine, Met, Ala, Val, Leu, and Ile;
(2) neutral hydrophilic: Cys, Ser, Thr, Asn, and Gln;
(3) acidic: Asp and Glu;
(4) basic: His, Lys, and Arg;
(5) residues that influence chain orientation: Gly and Pro;
(6) aromatic: Trp, Tyr, and Phe.

Non-conservative substitutions will entail exchanging a member of one of these classes for another class.

One type of substitutional variant involves substituting one or more hypervariable region residues of a parent antibody (e.g., a humanized antibody). Generally, the resulting variant(s) selected for further study will have modifications (e.g., improvements) in certain biological properties (e.g., increased affinity and reduced immunogenicity) relative to a parent antibody, and/or will have specific biological properties that are substantially retained by the parent antibody. An exemplary substitutional variant is an affinity-matured antibody, which can be conveniently produced by, for example, using phage-based affinity maturation techniques such as those described herein. In short, one or more HVR residues are mutated, and the variant antibodies are displayed on phages and screened for a particular biological activity (e.g., binding affinity).

The "percent (%) amino acid sequence identity" with respect to a reference polypeptide sequence is defined as the percentage of amino acid residues in a candidate sequence that are identical to the amino acid residues in the reference polypeptide sequence, after aligning the sequences and introducing gaps, if necessary, to achieve the maximum percent sequence identity, and not considering any conservative substitutions as part of the sequence identity. Alignment of sequences for the purpose of determining percent amino acid sequence identity can be achieved by using a variety of methods in the art, for instance, using publicly available computer software, such as BLAST, BLAST-2, ALIGN or MEGALIGN (DNASTAR) software. A person skilled in the art can determine appropriate parameters for measuring alignment, including any algorithms needed to achieve maximal alignment over the full length of the sequences being compared. When percentages of sequence identity are referred to in the present application, these percentages are calculated with respect to the full length of the longer sequence, unless otherwise specified. This calculation with respect to the full length of the longer sequence applies to both nucleic acid sequences and polypeptide sequences.

The terms "effective amount" and "therapeutically effective amount" refer to the amount or dose of the antibody of the present invention or the antigen-binding fragment, which upon administration to a patient in a single or multiple doses, produces the desired effect in a subject being treated, including the improvement in the condition of the subject (e.g., the improvement in one or more symptoms) and/or the delay in the progression of symptoms. The "effective amount" and "therapeutically effective amount" can also refer to an amount sufficient to reduce PD-L1 signaling.

The effective amount can be readily determined by the attending physician as a person skilled in the art with consideration of a variety of factors, such as the species of the mammal; the size, age and general health thereof; the specific disease involved; the extent or severity of the disease; the response of an individual patient; the specific antibody administered; the mode of administration; the bioavailability characteristics of the formulation administered; the selected dosing regimen; and use of any concomitant therapy.

The term "blocking" used herein indicates reducing PD-L1 signaling in the presence of the antibody of the present invention. Blocking of PD-L1-mediated signaling means that the PD-L1 signaling level in the presence of the PD-L1 single-domain antibody of the present invention is lower than the control PD-L1 level (namely, the PD-L1 signaling level in the absence of the antibody), with the reduction range greater than or equal to 5%, 10%, 15%, 20%, 25%, 30%, 35%, 40%, 45%, 50%, 55%, 60%, 65%, 70%, 75%, 80%, 85%, 90%, 95%, 99% or 100%. The PD-L1 signaling level can be measured by using a variety of standard techniques, such as, by way of non-limiting examples, luciferase reporter assays which measure the activation of the downstream gene and/or the activation in response to PD-L1. It is understood by a person skilled in the art that the PD-L1 signaling level can be measured by using a variety of assays, including, for example, commercially available kits.

The terms "host cell", "host cell line", and "host cell culture" are used interchangeably and refer to the cell into which exogenous nucleic acid is introduced, including the progeny of such cell. The host cell includes a "transformant" and a "transformed cell", which includes a primary transformed cell and the progeny derived therefrom regardless of the number of passages. The progeny may be not completely identical to the parent cell in terms of nucleic acid content, but may contain mutations. The mutant progeny that has the same function or biological activity screened or selected in the original transformed cell is included herein.

The term "vector" when used herein denotes a nucleic acid molecule capable of propagating another nucleic acid to which it is linked. The term includes the vector as a self-replicating nucleic acid structure and the vector incorporated into the genome of a host cell into which it has been introduced. Some vectors are capable of directing the expression of nucleic acids to which they are operatively linked. Such vectors are referred to herein as "expression vectors".

An "individual" or "subject" includes mammals. Mammals include, but are not limited to, domesticated animals (e.g., cattles, sheep, cats, dogs, and horses), primates (e.g., humans and non-human primates such as monkeys), rabbits, alpacas, and rodents (e.g., mice and rats). In some embodiments, the individual or subject is a human.

### II. PD-L1 single-domain antibodies or antigen-binding fragments thereof

The isolated PD-L1 binding molecule of the present invention can be a single-domain antibody or an antigen-binding fragment thereof. In other words, the present invention provides an isolated PD-L1 single-domain antibody or an antigen-binding fragment thereof, which is capable of specifically binding to PD-L1.

Therefore, the present invention also provides the following embodiments:
1. An isolated PD-L1 single-domain antibody or an antigen-binding fragment thereof, which specifically binds to PD-L1 and comprises a heavy chain variable region, wherein the heavy chain variable region comprises:
   (i) a CDR1 comprising the amino acid sequence of SEQ ID NO: 1;
      a CDR1 comprising an amino acid sequence having at least 80%, 85%, 90%, 95% or 99% identity to SEQ ID NO: 1; or
      a CDR1 comprising an amino acid sequence which differs from SEQ ID NO: 1 by no more than 2 (e.g., 0, 1 and 2) amino acid additions, deletions and/or substitutions;
   (ii) a CDR2 comprising the amino acid sequence of SEQ ID NO: 2;
      a CDR2 comprising an amino acid sequence having at least 80%, 85%, 90%, 95% or 99% identity to SEQ ID NO: 2; or
      a CDR2 comprising an amino acid sequence which differs from SEQ ID NO: 2 by no more than 2 (e.g., 0, 1, and 2) amino acid additions, deletions and/or substitutions;
         and
   (iii) a CDR3 comprising the amino acid sequence of SEQ ID NO: 3 or 12;
      a CDR3 comprising an amino acid sequence having at least 80%, 85%, 90%, 95% or 99% identity to SEQ ID NO: 3 or 12; or
      a CDR3 comprising an amino acid sequence which differs from SEQ ID NO: 3 or 12 by no more than 2 (e.g., 0, 1, and 2) amino acid additions, deletions and/or substitutions.
2. The isolated PD-L1 single-domain antibody or the antigen-binding fragment thereof according to embodiment 1, wherein the heavy chain variable region comprises:
   (i) a CDR1 as shown in formula RTDSNIX₁GMH, wherein X₁ is H, F or N;
   (ii) a CDR2 as shown in formula TIFIDX₂NTX₃, wherein X₂ is G, L or A, and X₃ is I or L; and
   (iii) a CDR3 as shown in formula DVSGYGRX₄, wherein X₄ is A or Y.
3. The isolated PD-L1 single-domain antibody or the antigen-binding fragment thereof according to embodiment 1, wherein the heavy chain variable region comprises:
   (i) a CDR1 comprising or consisting of SEQ ID NO: 1;
   (ii) a CDR2 comprising or consisting of SEQ ID NO: 2; and
   (iii) a CDR3 comprising or consisting of SEQ ID NO: 3.
4. The isolated PD-L1 single-domain antibody or the antigen-binding fragment thereof according to embodiment 1, wherein the heavy chain variable region comprises:
   (i) a CDR1 comprising or consisting of SEQ ID NO: 4;
   (ii) a CDR2 comprising or consisting of SEQ ID NO: 5; and
   (iii) a CDR3 comprising or consisting of SEQ ID NO: 3.
5. The isolated PD-L1 single-domain antibody or the antigen-binding fragment thereof according to embodiment 1, wherein the heavy chain variable region comprises:
   (i) a CDR1 comprising or consisting of SEQ ID NO: 6;
   (ii) a CDR2 comprising or consisting of SEQ ID NO: 7; and
   (iii) a CDR3 comprising or consisting of SEQ ID NO: 3.
6. The isolated PD-L1 single-domain antibody or the antigen-binding fragment thereof according to embodiment 1, wherein the heavy chain variable region comprises:
   (i) a CDR1 comprising or consisting of SEQ ID NO: 1;
   (ii) a CDR2 comprising or consisting of SEQ ID NO: 8; and
   (iii) a CDR3 comprising or consisting of SEQ ID NO: 9.
7. The isolated PD-L1 single-domain antibody or the antigen-binding fragment thereof according to embodiment 1, wherein the heavy chain variable region comprises:
   (i) a CDR1 comprising or consisting of SEQ ID NO: 10;
   (ii) a CDR2 comprising or consisting of SEQ ID NO: 11; and
   (iii) a CDR3 comprising or consisting of SEQ ID NO: 12.
8. The isolated PD-L1 single-domain antibody or the antigen-binding fragment thereof according to embodiment 1, wherein the heavy chain variable region comprises:
   (i) a CDR1 comprising or consisting of SEQ ID NO: 13;
   (ii) a CDR2 comprising or consisting of SEQ ID NO: 5; and
   (iii) a CDR3 comprising or consisting of SEQ ID NO: 3.
9. The isolated PD-L1 single-domain antibody or the antigen-binding fragment thereof according to embodiment 1, wherein the heavy chain variable region comprises:
   (i) a CDR1 comprising or consisting of SEQ ID NO: 14;
   (ii) a CDR2 comprising or consisting of SEQ ID NO: 15; and
   (iii) a CDR3 comprising or consisting of SEQ ID NO: 3.
10. The isolated PD-L1 single-domain antibody or the antigen-binding fragment thereof according to embodiment 1, wherein the heavy chain variable region comprises:
   (i) a CDR1 comprising or consisting of SEQ ID NO: 16;
   (ii) a CDR2 comprising or consisting of SEQ ID NO: 17; and
   (iii) a CDR3 comprising or consisting of SEQ ID NO: 3.
11. The isolated PD-L1 single-domain antibody or the antigen-binding fragment thereof according to embodiment 1, wherein the heavy chain variable region comprises:
   (i) a CDR1 comprising or consisting of SEQ ID NO: 18;
   (ii) a CDR2 comprising or consisting of SEQ ID NO: 19; and
   (iii) a CDR3 comprising or consisting of SEQ ID NO: 3.
12. The isolated PD-L1 single-domain antibody or the antigen-binding fragment thereof according to embodiment 1, wherein the heavy chain variable region comprises:
   (i) a CDR1 comprising or consisting of SEQ ID NO: 20;
   (ii) a CDR2 comprising or consisting of SEQ ID NO: 5; and
   (iii) a CDR3 comprising or consisting of SEQ ID NO: 3.
13. An isolated PD-L1 single-domain antibody or an antigen-binding fragment thereof, which specifically binds to PD-L1 and comprises a heavy chain variable region, wherein the heavy chain variable region comprises:
   (i) a CDR1 comprising or consisting of SEQ ID NO: 21;
   (ii) a CDR2 comprising or consisting of SEQ ID NO: 22; and
   (iii) a CDR3 comprising or consisting of SEQ ID NO: 23.
14. An isolated PD-L1 single-domain antibody or an antigen-binding fragment thereof, which specifically binds to PD-L1 and comprises a heavy chain variable region, wherein the heavy chain variable region comprises:
   (i) a CDR1 comprising or consisting of SEQ ID NO: 24;
   (ii) a CDR2 comprising or consisting of SEQ ID NO: 25; and
   (iii) a CDR3 comprising or consisting of SEQ ID NO: 3.
15. An isolated PD-L1 single-domain antibody or an antigen-binding fragment thereof, which specifically binds to PD-L1 and comprises a heavy chain variable region, wherein the heavy chain variable region comprises:
   (i) a CDR1 comprising or consisting of SEQ ID NO: 26;
   (ii) a CDR2 comprising or consisting of SEQ ID NO: 27; and
   (iii) a CDR3 comprising or consisting of SEQ ID NO: 23.
16. An isolated PD-L1 single-domain antibody or an antigen-binding fragment thereof, which specifically binds to PD-L1 and comprises a heavy chain variable region, wherein the heavy chain variable region comprises:
   (i) a CDR1 comprising or consisting of SEQ ID NO: 28;
   (ii) a CDR2 comprising or consisting of SEQ ID NO: 29; and
   (iii) a CDR3 comprising or consisting of SEQ ID NO: 23.
17. An isolated PD-L1 single-domain antibody or an antigen-binding fragment thereof, which specifically binds to PD-L1 and comprises a heavy chain variable region, wherein the heavy chain variable region comprises:
   (i) a CDR1 comprising or consisting of SEQ ID NO: 30;
   (ii) a CDR2 comprising or consisting of SEQ ID NO: 31; and
   (iii) a CDR3 comprising or consisting of SEQ ID NO: 23.
18. An isolated PD-L1 single-domain antibody or an antigen-binding fragment thereof, which specifically binds to PD-L1 and comprises a heavy chain variable region, wherein the heavy chain variable region comprises 3 CDRs in a heavy chain variable region amino acid sequence of any one of SEQ ID NOs: 36-52.
19. The isolated PD-L1 single-domain antibody or the antigen-binding fragment thereof according to any one of embodiments 1-18, wherein the heavy chain variable region further comprises an FR region comprising FR1, FR2, FR3 and FR4, and the FR1, FR2, FR3 and FR4 and CDR1, CDR2 and CDR3 are arranged alternately on the heavy chain variable region to form a structure of FR1-CDR1-FR2-CDR2-FR3-CDR3-FR4 from N-terminus to C-terminus.
20. The isolated PD-L1 single-domain antibody or the antigen-binding fragment thereof according to embodiment 19, wherein the FR region comprises:
   (a) an FR1 comprising the amino acid sequence of SEQ ID NO: 53;
      an FR1 comprising an amino acid sequence having at least 90%, 95% or 99% identity to SEQ ID NO: 53; or
      an FR1 comprising an amino acid sequence which differs from SEQ ID NO: 53 by no more than 2 (e.g., 0, 1 and 2) amino acid additions, deletions and/or substitutions;
   (b) an FR2 comprising the amino acid sequence of SEQ ID NO: 54;
      an FR2 comprising an amino acid sequence having at least 90%, 95% or 99% identity to SEQ ID NO: 54; or
      an FR2 comprising an amino acid sequence which differs from SEQ ID NO: 54 by no more than 2 (e.g., 0, 1 and 2) amino acid additions, deletions and/or substitutions;
   (c) an FR3 comprising the amino acid sequence of SEQ ID NO: 55;
      an FR3 comprising an amino acid sequence having at least 90%, 95% or 99% identity to SEQ ID NO: 55; or
      an FR3 comprising an amino acid sequence which differs from SEQ ID NO: 55 by no more than 2 (e.g., 0, 1 and 2) amino acid additions, deletions and/or substitutions;
         and
   (d) an FR4 comprising the amino acid sequence as shown in SEQ ID NO: 56;
      an FR4 comprising an amino acid sequence having at least 90%, 95% or 99% identity to SEQ ID NO: 56; or
      an FR4 comprising an amino acid sequence which differs from SEQ ID NO: 56 by no more than 2 (e.g., 0, 1, and 2) amion acid additions, deletions and/or substitutions.
21. The isolated PD-L1 single-domain antibody or the antigen-binding fragment thereof according to embodiment 19, wherein the FR region comprises:
   (a) an FR1 comprising the amino acid sequence of SEQ ID NO: 57;
      an FR1 comprising an amino acid sequence having at least 90%, 95% or 99% identity to SEQ ID NO: 57; or
      an FR1 comprising an amino acid sequence which differs from SEQ ID NO: 57 by no more than 2 (e.g., 0, 1 and 2) amino acid additions, deletions and/or substitutions;
   (b) an FR2 comprising the amino acid sequence of SEQ ID NO: 58;
      an FR2 comprising an amino acid sequence having at least 90%, 95% or 99% identity to SEQ ID NO: 58; or
      an FR2 comprising an amino acid sequence which differs from SEQ ID NO: 58 by no more than 2 (e.g., 0, 1 and 2) amino acid additions, deletions and/or substitutions;
   (c) an FR3 comprising the amino acid sequence of SEQ ID NO: 59;
      an FR3 comprising an amino acid sequence having at least 90%, 95% or 99% identity to SEQ ID NO: 59; or
      an FR3 comprising an amino acid sequence which differs from SEQ ID NO: 59 by no more than 2 (e.g., 0, 1 and 2) amino acid additions, deletions and/or substitutions;
         and
   (d) an FR4 comprising the amino acid sequence of SEQ ID NO: 60;
      an FR4 comprising an amino acid sequence having at least 90%, 95% or 99% identity to SEQ ID NO: 60; or
      an FR4 comprising an amino acid sequence which differs from SEQ ID NO: 60 by no more than 2 (e.g., 0, 1, and 2) amino acid additions, deletions and/or substitutions.
22. The isolated PD-L1 single-domain antibody or the antigen-binding fragment thereof according to embodiment 19, wherein the FR region comprises:
   (a) an FR1 comprising the amino acid sequence ofSEQ ID NO: 61;
      an FR1 comprising an amino acid sequence having at least 90%, 95% or 99% identity to SEQ ID NO: 61; or
      an FR1 comprising an amino acid sequence which differs from SEQ ID NO: 61 by no more than 2 (e.g., 0, 1 and 2) amino acid additions, deletions and/or substitutions;
   (b) an FR2 comprising the amino acid sequence of SEQ ID NO: 58;
      an FR2 comprising an amino acid sequence having at least 90%, 95% or 99% identity to SEQ ID NO: 58; or
      an FR2 comprising an amino acid sequence which differs from SEQ ID NO: 58 by no more than 2 (e.g., 0, 1 and 2) amino acid additions, deletions and/or substitutions;
   (c) an FR3 comprising the amino acid sequence of SEQ ID NO: 59;
      an FR3 comprising an amino acid sequence having at least 90%, 95% or 99% identity to SEQ ID NO: 59; or
      an FR3 comprising an amino acid sequence which differs from SEQ ID NO: 59 by no more than 2 (e.g., 0, 1 and 2) amino acid additions, deletions and/or substitutions;
         and
   (d) an FR4 comprising the amino acid sequence of SEQ ID NO: 60;
      an FR4 comprising an amino acid sequence having at least 90%, 95% or 99% identity to SEQ ID NO: 60; or
      an FR4 comprising an amino acid sequence which differs from SEQ ID NO: 60 by no more than 2 (e.g., 0, 1, and 2) amino acid additions, deletions and/or substitutions.
23. The isolated PD-L1 single-domain antibody or the antigen-binding fragment thereof according to any one of embodiments 1-22, wherein the heavy chain variable region comprises or consists of an amino acid sequence of any one of SEQ ID NOs: 36-52.
24. The isolated PD-L1 single-domain antibody or the antigen-binding fragment thereof according to any one of embodiments 1-22, wherein the heavy chain variable region comprises an amino acid sequence having at least 80%, 85%, 90%, 95% or 99% identity to an amino acid sequence of any one of SEQ ID NOs: 36-52 and retaining the ability to specifically bind to PD-L1.
25. The isolated PD-L1 single-domain antibody or the antigen-binding fragment thereof according to any one of embodiments 1-22, wherein the heavy chain variable region comprises an amino acid sequence having one or more additions, deletions and/or substitutions (e.g., conservative substitutions) of amino acids compared to an amino acid sequence of any one of SEQ ID NOs: 36-52 and retaining the ability to specifically bind to PD-L1, wherein the one or more amino acid additions, deletions and/or substitutions (e.g., conservative substitutions) are no more than five, preferably no more than three.
26. The isolated PD-L1 single-domain antibody or the antigen-binding fragment thereof according to any one of embodiments 1-25, wherein the PD-L1 binding molecule is a camelized antibody, a humanized antibody, an affinity-matured antibody or a druggability-modified molecule.
27. The isolated PD-L1 single-domain antibody or the antigen-binding fragment thereof according to any one of embodiments 1-26, wherein the heavy chain variable region is fused to an additional molecule, and the additional molecule is selected from an Fc domain of an immunoglobulin (e.g., IgG), an antibody, an antigen-binding fragment of the antibody, an antibody-drug conjugate, an antibody-like molecule, an antigen-binding fragment of the antibody-like molecule or a fluorescent protein.
28. The isolated PD-L1 single-domain antibody or the antigen-binding fragment thereof according to embodiment 27, wherein the heavy chain variable region is fused to an Fc domain of human IgG (e.g., human IgG1 or human IgG4).
29. An isolated nucleic acid molecule, comprising a nucleotide sequence encoding the isolated PD-L1 single-domain antibody or the antigen-binding fragment thereof according to any one of embodiments 1-28.
30. A vector, comprising the nucleic acid molecule according to embodiment 29.
31. A host cell, comprising the vector according to embodiment 30.
32. A pharmaceutical composition, comprising an effective amount of the PD-L1 single-domain antibody or the antigen-binding fragment thereof according to any one of embodiments 1-28 and a pharmaceutically acceptable carrier.
33. A method for preparing the PD-L1 single-domain antibody or the antigen-binding fragment thereof according to any one of embodiments 1-28, wherein the method comprises the following steps:
   - expressing the nucleotide sequence encoding the PD-L1 single-domain antibody or the antigen-binding fragment thereof according to any one of embodiments 1-28 in a host cell to produce the PD-L1 single-domain antibody or the antigen-binding fragment thereof; and
   - isolating the PD-L1 single-domain antibody or the antigen-binding fragment thereof from the host cell.
34. A method for preventing or treating a disease associated with PD-L1 in a subject, the method comprising administering a therapeutically effective amount of the PD-L1 single-domain antibody or the antigen-binding fragment thereof according to any one of embodiments 1-28, or administering an effective amount of a pharmaceutical composition comprising the PD-L1 single-domain antibody or the antigen-binding fragment thereof according to any one of embodiments 1-27 to the subject.
35. A method for modulating an immune response in a subject, comprising administering a therapeutically effective amount of the PD-L1 single-domain antibody or the antigen-binding fragment thereof according to any one of embodiments 1-28, or administering an effective amount of a pharmaceutical composition comprising the PD-L1 single-domain antibody or the antigen-binding fragment thereof according to any one of embodiments 1-28 to the subject, such that the immune response in the subject is modulated.
36. The method according to embodiment 34 or 35, wherein the subject is a mammal, preferably a mouse or a human, more preferably a human.
37. The method according to embodiment 36, wherein the disease associated with PD-L1 is selected from renal cell carcinoma, non-small cell lung cancer, bladder cancer, urothelial carcinoma, and microsatellite unstable solid tumor.
38. The method according to embodiment 34 or 35, wherein the method further comprises administering in combination an effective amount of a second drug to a subject, wherein the second drug is a chemotherapeutic agent, a radiotherapeutic agent, or a biomacromolecular drug for the treatment of a disease associated with PD-L1.
39. The method according to embodiment 38, wherein the biomacromolecular drug is, for example, various monoclonal antibody drugs (e.g., rituximab, cetuximab and trastuzumab) that attack tumor cells by T cell recognition.
40. A kit for preventing or treating a disease associated with PD-L1 in a subject, comprising a container, wherein the container comprises at least one PD-L1 single-domain antibody or the antigen-binding fragment thereof according to any one of embodiments 1-29.
41. The PD-L1 single-domain antibody or the antigen-binding fragment thereof according to any one of embodiments 1-29, for use in preventing or treating a disease associated with PD-L1 in a subject.
42. The PD-L1 single-domain antibody or the antigen-binding fragment thereof according to any one of embodiments 1-29, for use in modulating an immune response in a subject.
43. Use of the PD-L1 single-domain antibody or the antigen-binding fragment thereof according to any one of embodiments 1-29 in the preparation of a pharmaceutical composition for modulating an immune response in a subject.
44. Use of the PD-L1 single-domain antibody or the antigen-binding fragment thereof according to any one of embodiments 1-29 in the preparation of a pharmaceutical composition for preventing or treating a disease associated with PD-L1 in a subject.
45. The use according to embodiment 43 or 44, wherein the subject is a mammal, preferably a mouse or a human, more preferably a human.
46. The use according to embodiment 45, wherein the disease associated with PD-L1 is selected from renal cell carcinoma, non-small cell lung cancer, bladder cancer, urothelial carcinoma, and microsatellite unstable solid tumor.

### Example

The present invention, thus generally described, will be understood more readily by reference to the following Examples, which are provided by way of illustration and are not intended to be limiting of the present invention.

It is to be understood by a person skilled in the art that the reagents, plasmids, cells, etc., used in the following examples, unless otherwise noted, are all commercially available products.

### Example 1 Construction of cell lines stably expressing PD-L1

In this example, the present inventors construct CHO-s cells expressing human PD-L1, monkey PD-L1 and mouse PD-L1, respectively, and prepare a control antibody from Roche.

### 1.1 Preparation of a control antibody

The light chain and heavy chain gene sequences of the control antibody Atezolizumab (Roche) are subjected to complete gene synthesis (gene synthesis supplier: General Biol). The control antibody is expressed by using the ExpiCHO transient transfection and expression system (purchased from Thermo Fisher); the culture medium is the ExpiCHO^{™} Expression Medium (Gibco, A29100-01); and the transfection kit is the ExpiFectamine^{™} CHO Transfection Kit (Gibco, A29129).

The specific method is as follows: the ExpiCHO expression plasmids for the light chain and heavy chain genes of the Atezolizumab antibody are constructed by molecular cloning. One day prior to transfection, ExpiCHO cells (purchased from Gibco A29127) are passaged, and on the day of tranfection, 25 µg of the constructed plasmids (a plasmid mixture containing a light chain coding gene and a heavy chain coding gene in a mass ratio of 2:1) are mixed with a transfection reagent, then added dropwise to 25 ml of ExpiCHO cell cultures, fully mixed and incubated at 37°C for 18-22 hours. Then a feed culture medium is added according to the instruction in the kit, and after feeding, the cells are cultured at 32°C. On day 5 after transfection, a second feed culture medium is added, and the cells are cultured at 32°C for 10-12 days. Then the expressed cell suspension is centrifuged at a high speed to obtain a supernatant. The obtained supernatant is filtered through a 0.22 µm membrane filter and then purified by an affinity purification method using a Protein A/G affinity chromatography column. 100 mM glycine salt (pH 3.0) is used to elute the protein of interest followed by neutralization to pH 7.0 with 1 M Tris-HCI. A small amount of samples are taken, then identified by SDS-PAGE. The rest of the products are aliquoted and cryopreserved.

The method for preparing the control antibody KN035 (see SEQ ID NO: 35 in the sequence listing for the amino acid sequence) is as follows:
the complete gene sequences of the control antibody KN035 are subjected to complete gene synthesis, and the ExpiCHO expression plasmids for the genes of the KN035 antibody are constructed by molecular cloning. One day prior to transfection, ExpiCHO cells (purchased from Gibco A29127) are passaged. In a 25 ml cell culture, 25 µg of the constructed plasmids are mixed with a transfection reagent, and then transfected according to the above-mentioned method for preparing the control antibody-KN035.

### 1.2 Construction of stable cell lines

The recombinant vectors (plasmids) expressing the full-length proteins of human PD-L1 (gi number: NP_054862.1), mouse PD-L1 (gi number: NP_068693) and rhesus monkey PD-L1 (gi number: ABO33163.1) are constructed, respectively. The constructed plasmids are introduced into CHO-s cells (purchased from Thermo Fisher) and A375 melanoma cell line (ATCC, CRL-1619) by an electroporation method. The CHO-s cell line with high expression of PD-L1 proteins of the above-mentioned three species and the A375 cell line with high expression of human PD-L1 (PD-L1-A375) are obtained by screening, respectively.

### 1.2.1 Construction of plasmids expressing human PD-1 and PD-L1 proteins

The expression vectors containing the full-length protein gene sequences of human PD-L1, mouse PD-L1 and rhesus monkey PD-L1 are synthesized by gene synthesis techniques, respectively. After ligation, the expression vectors are introduced into *Escherichia coli.* The *Escherichia coli* monoclones are picked, and then sequenced to obtain the correct plasmid clones which are subjected to plasmid extraction and resequencing.

### 1.2.2 Construction of the CHO-s cell line expressing PD-1 and PD-L1 proteins

### 1.2.2.1 Electroporation

CHO-s cells are cultured and maintained in a CD-CHO serum-free culture medium (Gibco, 10743029). One day prior to electroporation, the cells are passaged to 5×10⁶/mL, and the next day, the constructed plasmids are introduced into the CHO-s cells by using an electroporation kit (Invitrogen, Neon^{™} Kit, MPK10096) and an electroporation apparatus (Invitrogen, NeonTM Transfection System, MP922947). The electroporated cells are added to 3 mL of CD-CHO culture medium and placed in a carbon dioxide incubator at 37°C for 48 hours.

### 1.2.2.2 Cell plating and culture

The electroporated CHO-s cells are plated at 2000 cells/well in a 96-well cell culture plate, and L-Methionine sulfoximine (MSX) (Millipore, GSS-1015-F) is added at a final concentration of 30 µM/MI. The culture volume is kept at 100 µL/well with 1×GS supplement (Sigma, 58672C). The cells are placed in a carbon dioxide incubator at 37°C for 10 days, and then 50 µL of culture medium containing 30 µM MSX and 1×GS supplement is added.

### 1.2.2.3 Clone identification and cell amplification and culture

The grown clones are picked and transferred to a 24-well cell culture plate. The cell lines are identified by an FACS method, and clones with high expression level are selected for amplification, culture and cryopreservation. The related FACS identification methods are as follows:
1) firstly, collecting 2×10⁵ of untransfected CHO-s cells and each transfected monoclonal CHO-s cells, centrifuging the collected cells at 300 g to remove the supernatant, and resuspending the cells in a 96-well round-bottom plate with 200 µL of the formulated FACS buffer (1×PBS+2% FBS);
2) centrifuging the 96-well round-bottom plate at 300 g for 5 min, and removing the supernatant;
3) adding anti-PD-Ll antibody diluents or negative control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL of FACS buffer to the corresponding wells and resuspending the cells by using a multi-channel pipette;
5) repeating step 4) twice, and centrifuging the cell mixed solution at 300 g to remove the supernatant;
6) adding a PE-labeled anti-human IgG Fc flow cytometry antibody (Abcam, ab98596), pipetting the cells repeatedly with a multi-channel pipette and incubating the cells at 4°C for 30 minutes;
7) centrifuging the mixture at 300 g to remove the supernatant, adding an FACS buffer and resuspending the cells; and
8) repeating step 7) twice, adding an FACS buffer to the wells (200 µL/well), resuspending the cells, and performing detection by a flow cytometer (Beckman, CytoFLEX AOO-1-1102).

### 1.2.3 Preparation of the A375 cell line expressing PD-L1

The PD-L1 high-expressing A375 cell line (PD-L1-A375) is prepared by using the same method as that of 1.2.2.1 for electroporation of CHO-s cells, and is used for the construction of the animal tumor model of the A375 cell line.

### Example 2 Animal immunization and serum titer detection

### 2.1 Animal immunization

A PD-L1 (NP_054862.1) ectodomain protein (Sino Biological Co., Ltd., 10084-H05H) is purchased to immunize 2 alpacas (Nanchang Dajia Technology Co., Ltd). Each alpaca is immunized with 500 µg of ectodomain protein each time, once every 2 weeks, for a total of 4 times.

### 2.2 Serum titer detection

After the completion of the alpaca immunization, the alpaca serum is taken for immune titer detection.

Immune titer determination is performed to determine the binding ability of immune serum to recombinant protein PD-L1 (Sino Biological Co., Ltd., 10084-H05H) by an ELISA method, and to determine the immune effect according to the titer of the bound antibodies.

The specific method is as follows:
2.2.1 Antigen coating: one day prior to the immune titer determination, the recombinant antigen protein PD-L1 is diluted with PBS to a final concentration of 2 µg/mL, thereby obtaining a diluent. 30 µl of the obtained diluent is added to each well of the ELISA plate and coated overnight at 4°C. On the day of the immune titer determination, the plate is rinsed three times with PBS, then blocked with PBST containing 5% skimmed milk for two hours at room temperature, and subsequently rinsed three times with PBS.
2.2.2 Serum dilution: in a different plate for dilution, the unimmunized negative serum and the immunized serum are diluted with PBS, with 200-fold dilution for the first well and a 3-fold gradient dilution for the following 7 wells.
2.2.3 Antibody binding: the diluted serum is added to the first ELISA plate, incubated at 37°C for 1 h, and washed twice with PBS. Subsequently, a secondary antibody (goat anti-camelid IgG antibody) (purchased from Nanjing Genscript Biotech Corporation) is added at 1:5000 dilution and incubated for 1 h.
2.2.4 Color development and reading: after the above-mentioned incubation, the plate is washed with PBS 3 times, a color developing solution is added and color development is performed for 5 minutes. A stopping solution is added, and then the plate is read at OD450 by a microplate reader (Molecular Devices, SpecterMax 190). The results are shown in Table 1.

**Table 1. Results of ELISA color development experiment at different dilution ratios**

| **Dilution ratio** | **Negative serum** | **NSY004** | **NSY005** |
|---|---|---|---|
| **1:2000** | **0.209** | **2.582** | **2.163** |
| **1:4000** | **0.133** | **2.286** | **2.216** |
| **1:8000** | **0.093** | **1.923** | **2.131** |
| **1:16000** | **0.052** | **1.817** | **1.868** |
| **1:32000** | **0.054** | **1.337** | **1.218** |
| **1:64000** | **0.048** | **1.048** | **0.792** |
| **1:128000** | **0.042** | **0.761** | **0.587** |
| **1:256000** | **0.048** | **0.404** | **0.473** |

The two columns NSY004 and NSY005 are the results of ELISA titer detection of serum diluted at different times from two immunized alpacas, and the negative serum is the ELISA experiment result of the serum from an unimmunized alpaca. According to the results in Table 1, the two alpacas have the immune titers (IgG titers) of 256000 and show good immune effect, which can be used for the construction of the immune antibody library in the next step.

### Example 3 Construction and preliminary screening of alpaca immune library

After the completion of the animal immunization, 50 mL of fresh alpaca blood is collected, and peripheral blood mononuclear cells (PBMC) are separated by a Ficoll-Paque density gradient separation solution (GE, 17144003S) for the construction of a phage-displayed alpaca immune library of an anti-human PD-L1 antibodies.

The specific method is as follows:
the collected alpaca blood is diluted with PBS at a ratio of 1:1 (v/v). 15 ml of the Ficoll-Paque density gradient separation solution is taken and slowly added to a 50 ml centrifuge tube. The centrifuge tube is tilted and 30 mL of diluted alpaca blood is added slowly along the tube wall, so that the two layers of liquid maintain a clear separation interface. The solution is centrifuged at 4°C for 20 min, with an acceleration of 3 and a deceleration of 0. After centrifugation, the entire solution is divided into four layers, with the top layer being a plasma mixture, the bottom layer being red blood cells and granulocytes, the middle layer being Ficoll-Paque PLUS, and the junction of the top and middle layers being a white and narrow zone mainly composed of PBMCs, i.e., a PBMC cell layer. PBMC cells in the middle are carefully pipetted into a new 50 mL centrifuge tube with a pipette. The PBMC cells are rinsed twice with PBS and horizontally centrifuged at 4°C, 1500 rpm for 10 min, and finally resuspended with 1.5 ml PBS and counted by a microscope.

RNA is extracted from the isolated PBMC cells, and the extracted RNA is reversely transcribed into cDNA by a reverse transcription kit (TaKaRa, 6210A). As the molecular form of an alpaca antibody has no light chain and has a heavy chain without CH1, the molecular form thereof is different from that of a common antibody. Therefore, firstly, two fragments of different sizes are obtained by using common primers designed prior to the VH and in the CH2 by PCR, and the smaller fragment of interest is recovered by gel extraction. Subsequently, by aligning the amino acid sequences of all common VHH germlines, germline-specific primers containing NcoI and NotI restriction enzyme sites at both ends are designed to amplify all the VHH genes by using the product recovered as the template. Finally, the gene fragment of the antibody of interest is inserted into the vector for phage display by double digestion and ligation. It need to be pointed out that the C-terminus of the VHH gene on the expression vector is fused to the GIII gene in the phage expression vector. The ligation product is recovered by a recovery kit (Omega, D6492-02), and finally transformed into competent *Escherichia coli* SS320 by an electroporation apparatus (Bio-Rad, MicroPulser), and the transformed *Escherichia coli* SS320 is plated onto a 2-YT solid plate with ampicillin resistance. To calculate the library size, 10 µl of the bacterial culture from the library is taken for 10-fold gradient dilution, with 2 µl of the bacterial culture in each dilution gradient spotted on the plate, and the clones formed on the plate are used to calculate the total number of all the clones formed by electroporation, thereby obtaining the library size. The library size of this immune library is 1×10⁹.

Based on the size of this library, the bacteria in an amount 10 times the library size (about 20 OD) are added to a fresh 2-YT liquid culture medium, and the amount of the culture medium added is adjusted so that the initial OD value of the bacterial culture diluent is 0.05. Cultures are placed at 37°C, 220 rpm and cultured to the log growth phase, and at this time, VSCM13 helper phages in an amount 50 times the number of the bacteria are added, fully mixed and allowed to stand for 30 min and then cultured at 37°C, 220 rpm for 1 h. The obtained culture is centrifuged at 10000 rpm for 5 min and have the culture medium changed to a carbenicillin/kanamycin dual resistant 2-YT culture medium, and cultured at 30°C, 220 rpm overnight. The next day, the resulting culture is centrifuged at 13000 g for 10 min, and the supernatant is precipitated by adding 20% PEG/NaCl solution to obtain phages representing the alpaca immune antibody library, which phages are used for anti-PD-Ll antibody screening following rinsing with PBS once.

A recombinant PD-L1 protein, and two methods (magnetic bead panning and immunotube panning) are used in phage panning. The specific method is as follows.

### 3.1 Magnetic bead panning

Panning is performed based on the binding of a biotin-labeled recombinant PD-L1 protein to avidin-coupled magnetic beads (purchased from Thermo Fisher, Cat. No. 11205D). Firstly, the recombinant human PD-L1 protein is labeled with biotin (for the biotin labeling method, please make reference to the instruction of the biotin protein labeling kit (Roche), Cat. No. 11418165001), and the biotin-labeled PD-L1 protein is incubated with magnetic beads, so that the PD-L1 protein is bound to the magnetic beads. The magnetic beads conjugated with the PD-L1 antigen and the nanobody-displaying phage library are incubated at room temperature for 2 hours and washed 6-8 times with PBST. Then the non-specifically adsorbed phages are removed, and trypsin (Gibco) is added and mixed gently for 20 min, so as to elute the nanobody-displaying phages specifically binding to the human PD-L1 protein. The eluted phages are used to infect SS320 bacterial cells at log-phase (Lucigen, MC1061 F), and the phage-infected SS320 bacterial cells are plated onto a carbenicillin-resistant plate and cultured at 37°C overnight. The bacterial cells are collected the next day. The phages are prepared by infecting SS320 bacterial cells. For the preparation method, please make reference to the above-mentioned library phage preparation method. The resulting phages are used for a second round of panning, and elution is performed by using trypsin at the end of the second round of panning. The phages obtained from the second round of panning are used for a third round of panning, and elution is performed by using trypsin at the end of the third round of panning. The procedures are repeated as such and sequence analysis is performed on 10 clones randomly selected from each round of panning. The results show that for the monoclonal phages obtained after 3 rounds of panning, it is found by sequencing of monoclones that in different clones, the same gene sequences are repeated, which indicates the sequence enrichment.

### 3.2 Immunotube panning

Immunotube panning is performed based on coating the antigen on the surface of an immunotube to screen for antibody-displaying phages that bind to the antigen of interest. One day prior to panning, the recombinant human PD-L1 protein is used to coat the immunotube. The immunotube conjugated with the PD-L1 antigen and the nanobody-displaying phage library are incubated at room temperature for 2 hours and washed 6-8 times with PBST. Then the non-specifically adsorbed phages are removed, and trypsin (Gibco) is added and mixed gently for 20 min, so as to elute the nanobody-displaying phages specifically binding to the human PD-L1 protein. The eluted phages are used to infect SS320 bacterial cells at log-phase (Lucigen, MC1061 F), and the phage-infected SS320 bacterial cells are plated onto a carbenicillin-resistant plate and cultured at 37°C overnight. The bacterial cells are collected the next day. The phages are prepared by infesting SS320 bacterial cells. For the preparation method, please make reference to the above-mentioned library phage preparation method. The resulting phages are used for a second round of panning. Elution is performed by using trypsin at the end of the second round of panning. The phages obtained from the second round of panning are used for a third round of panning, and elution is performed by using trypsin at the end of the third round of panning. The procedures are repeated as above. Sequencing is performed on 10 clones randomly selected from each round of panning, and then sequence analysis is performed thereon. The results show that after 3 rounds of panning, it is found by sequencing of monoclones that in different clones, the same gene sequences are repeated, which indicates the sequence enrichment.

The phage libraries obtained by two different panning methods are screened for monoclones, and the positive clones in the third round of outputs from magnetic bead panning and immunotube panning are picked, respectively. The specific method is as follows:
one day prior to screening, the recombinant human PD-L1 protein is coated on a 96-well ELISA plate, and the phage culture is incubated in the 96-well plate the next day. The positive clones against the recombinant human PD-L1 protein are screened by phage ELISA, and then all the positive clones are picked for sequencing analysis. Subsequently, a lysate is prepared with each clone having the unique sequence, and the preparation method is as follows: one day prior to preparation, the bacterial culture of the clone (50 mL) is inoculated at 1:100, and cultured with shaking in a 37°C constant-temperature shaker for 14 h. The culture medium is centrifuged at 10000 g for 5 min at room temperature. The bacteria are resuspended in 1 mL of Tris-HCl buffer containing benzonase nuclease at PH 9.0, lysed on ice for 30 min, and centrifuged at 10000 g for 10 min at 4°C. The supernatant is collected to obtain a positive clone lysate.

The prepared positive clone lysate is further verified by flow cytometry, and a candidate antibody that specifically recognizes human PD-L1 is screened out. The method for verifying by flow cytometry is as follows:
1) firstly, collecting the cultured human PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the PD-L1-CHO cells to a 96-well round-bottom plate at 100 µL/well, and centrifuging the cells at 300 g to remove the supernatant;
3) adding a gradient-diluted candidate antibody lysate and a control antibody diluent to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL of FACS buffer to the corresponding wells and resuspending the cells by using a multi-channel pipette;
5) repeating step 4) twice, and centrifuging the cell mixed solution at 300 g to remove the supernatant;
6) adding a PE-labeled flow cytometry antibody (Genscript Biotech Corporation), pipetting the cells repeatedly with a multi-channel pipette and incubating the cells at 4°C for 30 minutes;
7) centrifuging the mixture at 300 g to remove the supernatant, adding an FACS buffer and resuspending the cells; and
8) repeating step 7) twice, adding an FACS buffer to the wells (200 µL/well), resuspending the cells, and performing detection by a flow cytometer (Beckman, CytoFLEX AOO-1-1102).

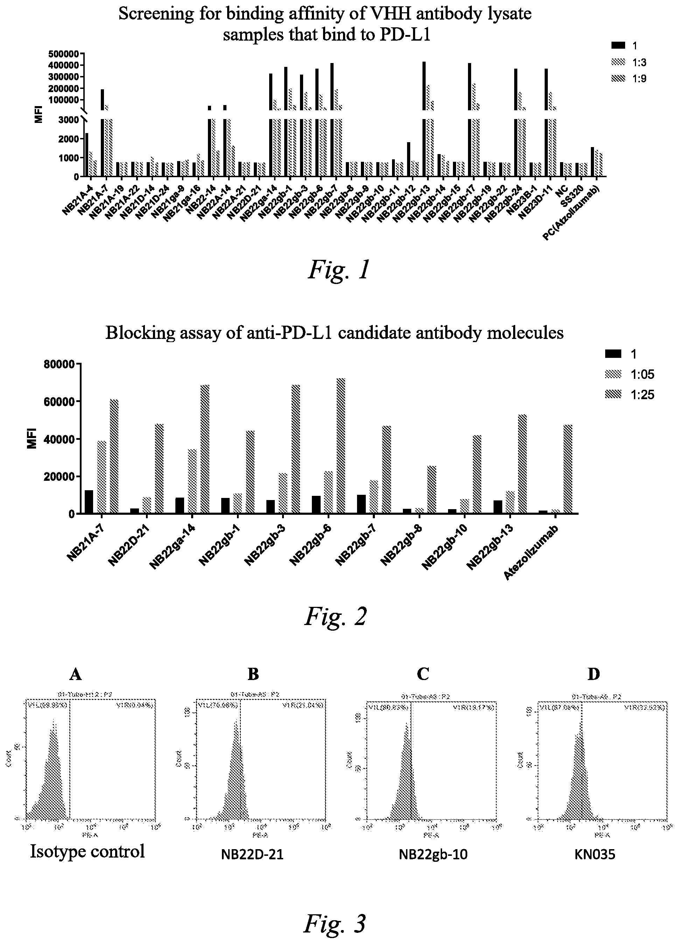

Figure 1 shows the results of screening for binding affinity of anti-PD-L1 VHH lysate samples.

Figure 1 shows that the VHH molecules of the indicated clone numbers exhibit affinity for CHO cells expressing PD-L1. Since this detection experiment is only qualitative and semi-quantitative, it is impossible to confirm which VHH antibody molecule of the clone number has better affinity, and further experimental confirmation is required.

The prepared positive clone lysate is further screened for blocking activity by flow cytometry, and a candidate antibody that specifically recognizes human PD-L1 and blocks the binding thereof to a PD-1 protein is screened out. The method for verifying the blocking activity by flow cytometry is as follows:
1) firstly, collecting the cultured human PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the PD-L1-CHO cells to a 96-well round-bottom plate at 100 µL/well, and centrifuging the cells at 300 g to remove the supernatant;
3) adding the candidate antibody lysates and the control antibody diluents with concentration gradients of 1:1, 1:5 and 1:25 (obtained by gradient dilution) to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding 100 µL of PD-1-Fc protein diluent (1 µg/mL) to the corresponding wells, resuspending the cells and incubating the cells at 4°C for 30 minutes;
7) centrifuging the incubated cell mixed solution at 300 g to remove the supernatant, adding 200 µL FACS buffer to the corresponding wells and resuspending the cells by using a multi-channel pipette;
8) repeating step 7) twice, and centrifuging the cell mixed solution at 300 g to remove the supernatant;
9) adding a PE-labeled anti-human IgG Fc flow cytometry antibody (Abcam), pipetting the cells repeatedly with a multi-channel pipette and incubating the cells at 4°C for 30 minutes;
10) centrifuging the mixture at 300 g to remove the supernatant, adding an FACS buffer and resuspending the cells; and
11) repeating step 10) twice, adding an FACS buffer to the wells (200 µL/well), resuspending the cells, and performing detection by a flow cytometer (Beckman, CytoFLEX AOO-1-1102).

The screening results of the blocking assay of anti-PD-Ll antibody candidates are shown in Figure 2. By means of the preliminary screening by methods such as ELISA and FACS, it shows that the antibody lysate prepared from the screened antibody sequences contains antibodies that have the blocking activity against the binding of PD-L1 to PD1. The present inventors screen out 10 candidate molecules with good affinity and blocking activity.

### Example 4 Expression and production of a chimeric VHH-Fc antibody

The positive VHH candidate antibodies obtained by screening is fused to the human IgG1 Fc fragment by molecular cloning. The C-terminus of the positive VHH gene sequence is linked to the N-terminus of the human IgG1 Fc fragment gene sequence to construct a fusion expression plasmid. The fusion expression plasmid is transformed into ExpiCHO cells to induce expression, thereby obtaining a VHH-Fc chimeric antibody protein.

The antibody is expressed by using the ExpiCHO transient transfection and expression system; the culture medium is the medium of Gibco ExpiCHO Expression Medium, A29100-01; and the transfection kit is ExpiFectamine^{™} CHO Transfection Kit, A29129. The specific method is as follows: one day prior to transfection, ExpiCHO cells are passaged, and in a 25 ml culture, 25 µg of the constructed plasmids are mixed with the transfection reagent, then added dropwise to 25 ml of ExpiCHO cell cultures, fully mixed and expressed at 37°C for 18-22 hours. Then a feed culture medium is added according to the instruction in the kit, and after feeding, the cells are cultured at 32°C. On day 5 after transfection, a second feed culture medium is added, and the cells are cultured at 32°C for 10-12 days. Then the expressed cell suspension is centrifuged at a high speed to obtain a supernatant. The obtained supernatant is filtered through 0.22 µm and then purified by using a Protein A/G affinity purification method. 100 mM glycine salt (pH 3.0) is used to elute the protein of interest followed by neutralization with 1 M Tris-HCl.

### Example 5 Verification of affinity activity of the chimeric VHH-Fc antibodies in vitro

In order to evaluate the obtained VHH-Fc candidate antibodies, an FACS method is used to detect the binding activity thereof to the PD-L1 protein expressing cells. The specific method is as follows:
1) collecting the cultured human PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the PD-L1-CHO cells to a 96-well round-bottom plate at 100 µL/well, and centrifuging the cells at 300 g to remove the supernatant;
3) adding the gradient-diluted candidate antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding a PE-labeled anti-human IgG Fc flow cytometry antibody (Abcam, ab98596), pipetting the cells repeatedly with a multi-channel pipette and incubating the cells at 4°C for 30 minutes;
7) centrifuging the mixture at 300 g to remove the supernatant, adding the FACS buffer and resuspending the cells; and
8) repeating step 10) twice, adding the FACS buffer to the wells (200 µL/well), resuspending the cells, and performing detection by a flow cytometer (Beckman, CytoFLEX AOO-1-1102).

As shown in Table 2, by the FACS experiment, the present inventors screen out 2 nanobody candidates with high affinities (NB22D-21 and NB22gb-10), and the affinities thereof are higher than or similar to that of the control antibody.

**Table 2. EC50 of antibodies**

| **Clone number** | **EC50 (µg/mL)** |
|---|---|
| **NB22D-21** | **0.39** |
| **NB22gb-10** | **0.39** |
| **KN035 (control)** | **0.39** |
| **Atezolizumab (control)** | **0.83** |

### Example 6 Verification of blocking activity of the chimeric VHH-Fc antibodies against PD-1

In order to evaluate the obtained VHH-Fc candidate antibodies, an FACS method is used to detect the blocking activity thereof against PD-1/PD-L1. The specific method is as follows:
1) collecting the cultured human PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the PD-L1-CHO cells to a 96-well round-bottom plate at 100 µL/well, and centrifuging the cells at 300 g to remove the supernatant;
3) adding the gradient-diluted candidate antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding 100 µL of biotin-labeled PD-1-Fc protein diluent (1 µg/mL) to the corresponding wells, resuspending the cells and incubating the cells at 4°C for 30 minutes;
7) centrifuging the incubated cell mixed solution at 300 g to remove the supernatant, adding 200 µL FACS buffter to the corresponding wells and resuspending the cells by using a multi-channel pipette;
8) repeating step 7) twice, and centrifuging the cell mixed solution at 300 g to remove the supernatant;
9) adding a PE-labeled streptavidin (eBioscience, 12-4317-87), pipetting the cells repeatedly with a multi-channel pipette and incubating the cells at 4°C for 30 minutes;
10) centrifuging the mixture at 300 g to remove the supernatant, adding an FACS buffer and resuspending the cells; and
11) repeating step 10) twice, adding the FACS buffer to the wells (200 µL/well), resuspending the cells, and performing detection by a flow cytometer (Beckman, CytoFLEX AOO-1-1102).

As shown in Table 3, by the FACS experiment, the present inventors verify that the 2 nanobody candidates with clone numbers NB22D-21 and NB22gb-10 in Example 5 both have high blocking activities, and the blocking activities thereof are higher than or similar to that of the control antibody.

**Table 3. IC50 of antibodies**

| Clone number | IC50 (µg/mL) |
|---|---|
| NB22D-21 | 0.41 |
| NB22gb-10 | 0.54 |
| KN035 (control) | 0.39 |
| Atezolizumab (control) | 0.86 |

### Example 7 Verification of blocking activity of the chimeric VHH-Fc antibodies against CD80

In order to evaluate the obtained VHH-Fc candidate antibodies, an FACS method is used to detect the blocking activity thereof against CD80/PD-L1. The specific method is as follows:
1) collecting the cultured human PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the PD-L1-CHO cells to a 96-well round-bottom plate at 100 µL/well, and centrifuging the cells at 300 g to remove the supernatant;
3) adding the gradient-diluted candidate antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding 100 µL of biotin-labeled CD80 protein diluent (1 µg/mL) to the corresponding wells, resuspending the cells and incubating the cells at 4°C for 30 minutes;
7) centrifuging the incubated cell mixed solution at 300 g to remove the supernatant, adding 200 µL FACS buffer to the corresponding wells and resuspending the cells by using a multi-channel pipette;
8) repeating step 7) twice, and centrifuging the cell mixed solution at 300 g to remove the supernatant;
9) adding a PE-labeled streptavidin (eBioscience, 12-4317-87), pipetting the cells repeatedly with a multi-channel pipette and incubating the cells at 4°C for 30 minutes;
10) centrifuging the mixture at 300 g to remove the supernatant, adding the FACS buffer and resuspending the cells; and
11) repeating step 10) twice, adding the FACS buffer to the wells (200 µL/well), resuspending the cells, and performing detection by a flow cytometer (Beckman, CytoFLEX AOO-1-1102).

As shown in Table 4, by the FACS experiment, the present inventors verify that the 2 nanobody candidate molecules with clone numbers NB22D-21 and NB22gb-10 in Example 5 both have high blocking activities, and the blocking activities thereof are higher than or similar to that of the control antibody.

**Table 4. IC50 of antibodies**

| Clone number | IC50 (µg/mL) |
|---|---|
| NB22D-21 | 0.7081 |
| NB22gb-10 | 0.7651 |
| KN035 (control) | 0.6307 |
| Atezolizumab (control) | 0.9077 |

### Example 8 Binding activity of the chimeric VHH-Fc antibodies on tumor cells

In order to evaluate the binding activity of the VHH-Fc candidate antibodies on human melanoma cell line A375 cells (ATCC, CRL-1619), an FACS method is used to detect the binding activity thereof to the PD-L1 protein on A375 cells. The specific method is as follows:
1) detaching A375 cells with trypsin containing 0.25% EDTA, collecting the cells, centrifuging the collected cells at 300 g to remove the supernatant, resuspending the cells in a formulated FACS buffer and counting the cells, and adjusting the density of the cell suspension to 2×10⁶ cells/mL;
2) adding the A375 cells to a 96-well round-bottom plate at 100 µL/well, and centrifuging the cells at 300 g to remove the supernatant;
3) adding the gradient-diluted candidate antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding 100 µL of diluents of each candidate antibody and the control antibody (1 µg/mL) to the corresponding wells, resuspending the cells and incubating the cells at 4°C for 30 minutes;
7) centrifuging the incubated cell mixed solution at 300 g to remove the supernatant, adding 200 µL to the corresponding wells and resuspending the cells by using a multi-channel pipette;
8) repeating step 7) twice, and centrifuging the cell mixed solution at 300 g to remove the supernatant;
9) adding a PE-labeled anti-biotin flow cytometry antibody (Abcam), pipetting the cells repeatedly with a multi-channel pipette and incubating the cells at 4°C for 30 minutes;
10) centrifuging the mixture at 300 g to remove the supernatant, adding the FACS buffer and resuspending the cells; and
11) repeating step 10) twice, adding an FACS buffer to the wells (200 µL/well), resuspending the cells, and performing detection by a flow cytometer (Beckman, CytoFLEX AOO-1-1102) (the detection results are shown in Figure 3).

Figure 3 shows that the 2 nanobody candidates with clone numbers NB22D-21 and NB22gb-10 have comparable binding activity on human melanoma cell line A375 cells to that of the control antibody.

### Example 9 Verification of binding activity of the chimeric VHH-Fc antibodies to mouse PD-L1 and rhesus monkey PD-L1

In order to evaluate the cross-reactivity of the VHH-Fc candidate antibodies to monkey PD-L1 and mouse PD-L1, an FACS method is used to detect the binding activity thereof to the mouse and rhesus monkey PD-L1 protein on cells. The specific method is as follows:
1) collecting the cultured mouse PD-L1-CHO cells and monkey PD-L1-CHO cells, centrifuging the collected cells at 300 g to remove the culture medium, resuspending the cells in a formulated FACS buffer and counting the cells, and adjusting the density of the cell suspension to 2×10⁶ cells/mL;
2) adding the mouse PD-L1-CHO cells and the monkey PD-L1-CHO cells to a 96-well round-bottom plate at 100 µL/well, respectively, and centrifuging the cells at 300 g to remove the supernatant;
3) adding the gradient-diluted candidate antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding 100 µL of biotin-labeled PD-1-Fc protein diluent (1 µg/mL) to the corresponding wells, resuspending the cells and incubating the cells at 4°C for 30 minutes;
7) centrifuging the incubated cell mixed solution at 300 g to remove the supernatant, adding 200 µL to the corresponding wells and resuspending the cells by using a multi-channel pipette;
8) repeating step 7) twice, and centrifuging the cell mixed solution at 300 g to remove the supernatant;
96) adding a PE-labeled anti-biotin flow cytometry antibody (Abcam), pipetting the cells repeatedly with a multi-channel pipette and incubating the cells at 4°C for 30 minutes;
7) centrifuging the mixture at 300 g to remove the supernatant, adding the FACS buffer and resuspending the cells; and
8) repeating step 7) twice, adding the FACS buffer to the wells (200 µL/well), resuspending the cells, and performing detection by a flow cytometer (Beckman, CytoFLEX AOO-1-1102).

The detection results of the human-mouse cross reactivity show that the nanobody candidate molecule with clone number NB22D-21 has a certain binding activity to mouse PD-L1.

For the detection results of the human-monkey cross reactivity, Table 5 shows that the 2 nanobody candidates with clone numbers NB22D-21 and NB22gb-10 both have good activities in recognizing monkey PD-L1.

**Table 5. EC50 value**

| Clone number | EC50 (µg/mL) |
|---|---|
| NB22D-21 | 0.25 |
| NB22gb-10 | 0.20 |
| KN035 (control) | 0.21 |
| Atezolizumab (control) | 0.33 |

### Example 10 Specificity detection of the chimeric VHH-Fc antibodies to PD-L1

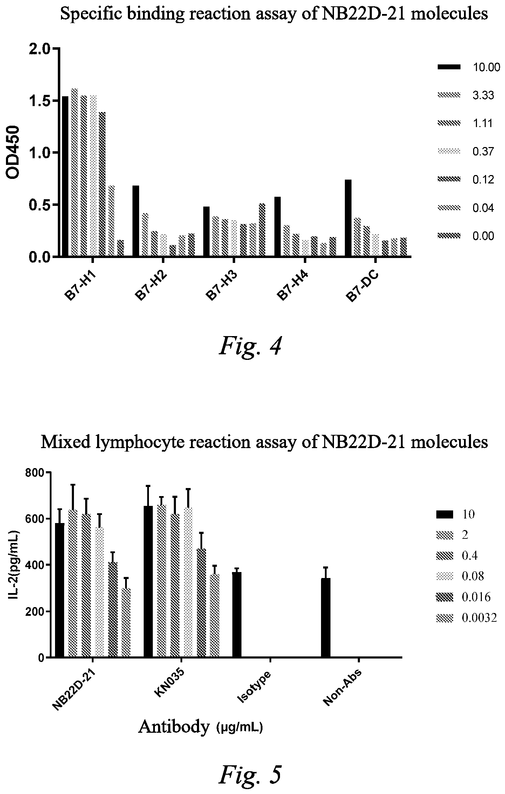

In order to confirm the binding specificity of the candidate antibodies to a PD-L1 protein, an ELISA method is used to detect the binding activity thereof to other proteins of the B7 family. The specific method is as follows:
one day prior to the experiment, 30 µL of protein diluents such as B7-H1 (i.e., PDL1), B7-H2, B7-H3, B7-H4 and B7-DC (the above-mentioned proteins are all purchased from Sino Biological Co., Ltd., with Cat. Nos. 10084-HNAH, 11559-H08H, 11188-H08H, 10738-H08H and 10292-H08H-B, respectively) are added to an ELISA plate at a final concentration of 2 µg/mL, and incubated at 4°C overnight; the next day, the ELISA plate is rinsed 3 times with PBST, then blocked with 150 µL of 5% PBSM, and incubated at room temperature for 2 hours; the ELISA plate is rinsed 3 times with PBST again, and then 30 µL of candidate antibody and control antibody diluents are added thereto, and the plate is incubated at room temperature for 1 hour; the plate is rinsed 3 times with PBST, and an anti-human IgG Fc-HRP second antibody diluted at 1:7000 is added thereto (30 µL/well), and the plate is incubated at room temperature for 30 minutes; the plate is rinsed 6 times with PBST, then TMB is added thereto for color development, and finally, 2M HCl is added to stop the reaction. The values are read at the wavelength of OD450 by using a microplate reader (Molecular Devices, SpecterMax 190), and the results are shown in Table 6 and Figure 4.

The results of the specificity assay are shown in Table 6 and Figure 4.

**Table 6. Binding specificity of candidate antibodies to the PD-L1 family proteins**

| Clone/control | B7-H1 | B7-H2 | B7-H3 | B7-H4 | B7-DC |
|---|---|---|---|---|---|
| NB22D-21 | + | - | - | - | - |
| NB22-gb-10 | + | - | - | - | - |
| KN035 | + | - | - | - | - |
| Atezolizumab | + | - | - | - | - |

| | | | | | |
|---|---|---|---|---|---|
| +: indicates the binding activity being detected. -: indicates no binding activity being detected. | | | | | |

Table 6 and Figure 4 showed that the 2 nanobody candidates with clone numbers NB22D-21 and NB22gb-10 have no binding activities to B7 family proteins other than B7-H1, but only have the binding activity to B7-H1, and this binding specificity is consistent with that of the control antibodies.

### Example 11 Verification of the in vitro biological activity of the chimeric VHH-Fc antibodies

In order to confirm the ability of the candidate antibody to stimulate T cell activation *in vitro,* the present inventors established a mixed lymphocyte reaction *in vitro* assay. The specific method is as follows:
CD14-positive monocytes and CD4-positive T cells are sorted *in vitro* by using a Miltenyibiotec sorting kit (Miltenyibiotec, 130-050-201). The monocytes are induced into dendritic cells (DCs) by *in vitro* induction culture with 100 ng/mL IL-4 and 100 ng/mL GM-CSF for 7 days. Then the CD4-positive T cells and DCs are mixed at a cell number ratio of 10:1, and the gradient-diluted candidate antibody and the control antibody are added to the cells and cultured in a cell incubator at 37°C for 5 days. After 72 hours of culture, the cell supernatant is taken and diluted with PBS, so as to detect the secretion amount of IL-2 in the culture supernatant by using an IL-2 ELISA kit; and after 5 days of culture, the cell supernatant is taken and diluted with PBS, so as to detect the secretion amount of IFN-γ in the culture supernatant by using an IFN-γ ELISA kit.

By detecting the secretion amount of IL-2, the present inventors obtain the verification results of mixed lymphocyte reaction assay of the candidate antibody, which results are shown in Figure 5. Figure 5 shows that the nanobody candidate of the present invention can activate the T cell immune response.

### Example 12 Humanization of the candidate antibody

In order to reduce the potential immunogenicity of the candidate, the present inventors perform humanization conversion on the candidate antibodies. Structural Modeling is performed by using a homology modeling method with Discovery Studio and Schrödinger Antibody Modeling, respectively, and 5-10 optimal structural solutions are selected. The homology modeling method is generally used to perform modeling on a Loop region, and if the CDR amino acid sequence alignment results show that the identity is lower than 50%, the de novo modeling method is used to build a CDR3 structural model. PDB BLAST is used to retrieve 10 antibody crystal structure models with the highest sequence identity (the structure resolution is higher than 2.5 angstroms); the models are compared with that from the automatic modeling, and the optimal structural model is selected. The present inventors preform antibody humanization modification on the NB22D-21 molecule, thus obtaining two humanized molecules, the numbers of which are NB22D-21-huVH1 and NB22D-21-huVH2, respectively.

### Example 13 Binding of the humanized candidate antobodies to the human PD-L1-CHO cells

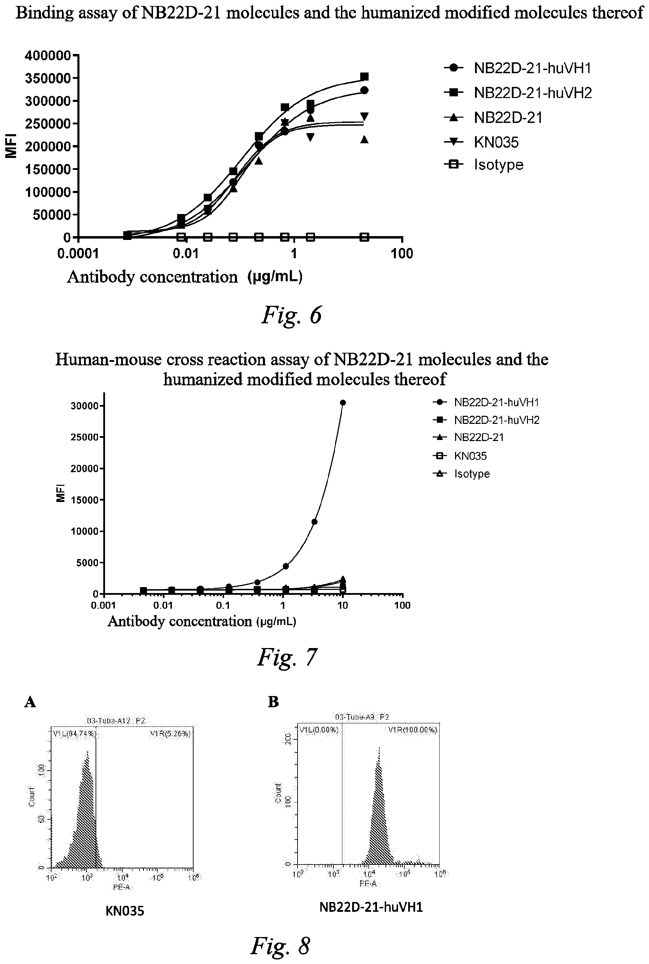

In order to detect the effect of antibody humanization on the binding activity to the human PD-L1 antigen, the present inventors use FACS to detect the parental antibody and the humanized antibodies thereof, as follows:
1) collecting the cultured human PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the PD-L1-CHO cells to a 96-well round-bottom plate at 100 µL/well, and centrifuging the cells at 300 g to remove the supernatant;
3) adding the gradient-diluted parental antibody and humanized antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding a PE-labeled anti-human IgG Fc flow cytometry antibody (Abcam, ab98596), pipetting the cells repeatedly with a multi-channel pipette and incubating the cells at 4°C for 30 minutes;
7) centrifuging the mixture at 300 g to remove the supernatant, adding the FACS buffer and resuspending the cells; and
8) repeating step 7) twice, adding the FACS buffer to the wells (200 µL/well), resuspending the cells, and performing detection by a flow cytometer (Beckman, CytoFLEX AOO-1-1102) (the results are shown in Figure 6).

Figure 6 shows that the humanized antibodies (i.e., NB22D-21-huVH1 and NB22D-21-huVH2) bind to human PD-L1 with the similar affinity as that of the parental antibody (i.e., NB22D-21).

### Example 14 Binding of the humanized antibodies to mouse PD-L1-CHO cells

In order to detect the effect of the humanization on the cross reactivity to the mouse PD-L1 antigen, the present inventors use FACS to detect the candidate antibodies.
1) collecting the cultured mouse PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the mouse PD-L1-CHO cells to a 96-well round-bottom plate at 100 µL/well, and centrifuging the cells at 300 g to remove the supernatant;
3) adding the gradient-diluted parental antibody and humanized antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding a PE-labeled anti-human IgG Fc flow cytometry antibody (Abcam, ab98596), pipetting the cells repeatedly with a multi-channel pipette and incubating the cells at 4°C for 30 minutes;
7) centrifuging the mixture at 300 g to remove the supernatant, adding the FACS buffer and resuspending the cells; and
8) repeating step 7) twice, adding the FACS buffer to the wells (200 µL/well), resuspending the cells, and performing detection by a flow cytometer (Beckman, CytoFLEX AOO-1-1102) (see Figure 7 and Figure 8 for results).

Figure 7 shows that among the humanized antibodies, NB22D-21-huVH1 binds to mouse PD-L1 with better affinity than that of the parent molecule (i.e., NB22D-21).

Specifically, it is shown in Figure 7 that compared with the parental antibody NB22D-21 and the control antibody KN035, the humanized antibody NB22D-21-huVH1 has a very good binding activity to the mouse PD-L1 protein; the peak plots in flow cytometry of KN035 and NB22D-21-huVH1 are selected (as shown in Figure 8), and it shows that when 20 µg/mL of KN035 and NB22D-21-huVH1 are used to bind to mouse PD-L1-overexpressing cells, respectively, KN035 cannot recognize mouse PD-L1 on the surface of the CHO cells (Figure 8A), while NB22D-21-huVH1 can recognize mouse PD-L1 on the surface of the CHO cells well (Figure 8B) (note: the dividing line of each figure is the position of the set fluorescence intensity threshold).

### Example 15 Verification of the blocking activity of the humanized antibodies against PD-1

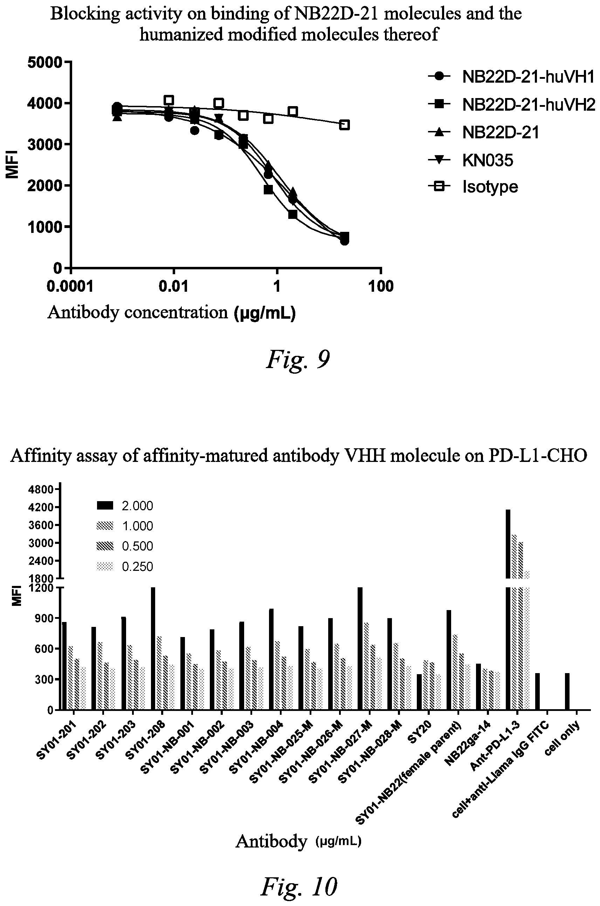

In order to evaluate the humanized antibodies, an FACS method is used to detect the blocking activity thereof against PD-1/PD-L1. The specific method is as follows:
1) collecting the cultured human PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the PD-L1-CHO cells to a 96-well round-bottom plate at 100 µL/well, and centrifuging the cells at 300 g to remove the supernatant;
3) adding the gradient-diluted parental antibody and humanized antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding 100 µL of biotin-labeled PD-1-Fc protein diluents (1 µg/mL) to the corresponding wells, resuspending the cells and incubating the cells at 4°C for 30 minutes;
7) centrifuging the incubated cell mixed solution at 300 g to remove the supernatant, adding 200 µL FACS buffer to the corresponding wells and resuspending the cells by using a multi-channel pipette;
8) repeating step 7) twice, and centrifuging the cell mixed solution at 300 g to remove the supernatant;
9) adding a PE-labeled streptavidin (eBioscience, 12-4317-87), pipetting the cells repeatedly with a multi-channel pipette and incubating the cells at 4°C for 30 minutes;
10) centrifuging the mixture at 300 g to remove the supernatant, adding the FACS buffer and resuspending the cells; and
11) repeating step 10) twice, adding the FACS buffer to the wells (200 µL/well), resuspending the cells, and performing detection by a flow cytometer (Beckman, CytoFLEX AOO-1-1102) (the results are shown in Figure 9).

Figure 9 shows that the humanized antibodies (i.e., NB22D-21-huVH1 and NB22D-21-huVH2) have comparable blocking activity against PD-1 protein binding to PD-L1 compared to that of the parent antibody (i.e., NB22D-21) and the positive control KN035.

### Example 16 Affinity maturation of the humanized antibodies

In order to improve the affinity and blocking activity of antibody molecules in binding to a human PD-L1 antigen, the present inventors perform affinity maturation to the humanized candidate.

Affinity maturation is performed by using methods such as single-site saturation mutagenesis, multi-points combinatorial mutagenesis and strand displacement; a Trim primer technique is used to directionally design primers for the CDR regions, thereby constructing an affinity-matured antibody library; and a phage display technique is used to screen the affinity-matured library. The VHH lysates are prepared after monoclones are obtained, and FACS is used to detect the affinity and blocking activity of the clones. By means of screening, we obtain 4 clones that may be superior to the parental, namely SY01-201, SY01-208, SY01-NB-004 and SY01-NB-027-M, respectively.

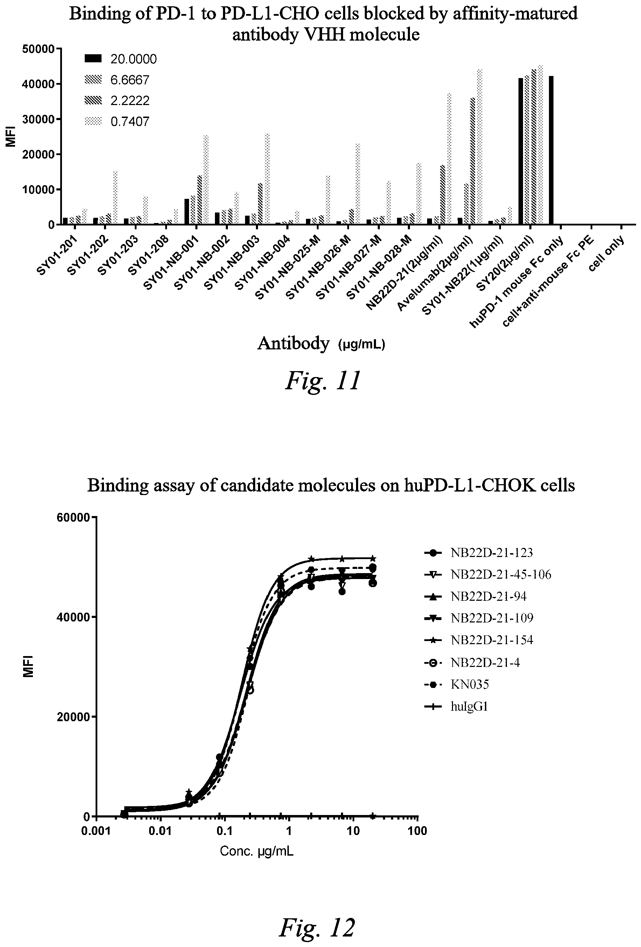

The specific experimental results are shown in Figure 10 and Figure 11. From the qualitative/semi-quantitative results of the monoclonal cell lysates, it indicates that the four molecules SY01-201, SY01-208, SY01-NB-004 and SY01-NB-027-M have similar binding activity and blocking activity to that of the parental antibody NB22D-21.

### Example 17 Engineering of the post-tranlational modification of the humanized anitobodies

In order to optimize the developability of the humanized antibody and avoid the influence of potential post-translational modification sites on protein folding, activity and functions, the present inventors perform antibody engineering design on the humanized antibody NB22D-21-huVH2.

Antibody engineering is performed by using point mutagenesis; random mutagenesis is performed on the potential post-translational modification sites, thereby constructing a derisked antibody library; and a phage display technique is used to screen the library. The VHH lysates are prepared after monoclones are obtained, and FACS is performed to detect the blocking activity of the clones with PD-L1-CHO cells.

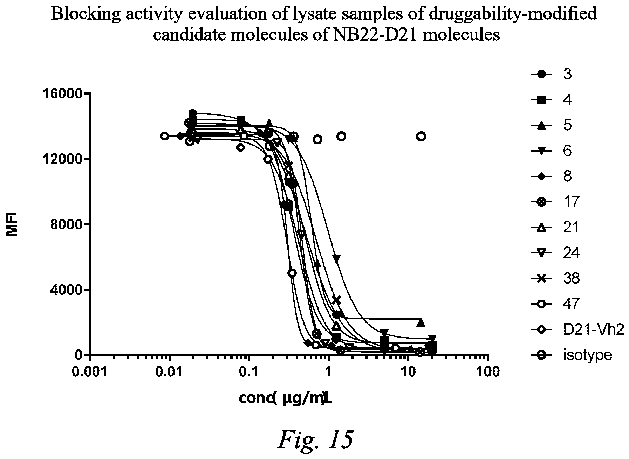

By means of preliminary screening, the present inventors obtain 10 deriksed candidate antibodies: SY01-D21-3, SY01-D21-4, SY01-D21-5, SY01-D21-6, SY01-D21-8, SY01-D21-17, SY01-D21-21, SY01-D21-24, SY01-D21-38 and SY01-D21-47 (respectively abbreviated as 3, 4, 5, 6, 8, 17, 21, 24, 38 and 47 in Figure 15; the parent molecule being NB22D-21-huVH2, abbreviated as D21-Vh2; and the isotype control being human IgG1), and by means of further screening, the present inventors obtain 5 clones that may be superior to the parental, namely SY01-D21-4, SY01-D21-8, SY01-D21-17, SY01-D21-24 and SY01-D21-47, respectively.

The experimental results are shown in Figure 15. From the qualitative/semi-quantitative results of the cell lysates of the candidates, it shows that the 4 modified candidates SY01-D21-4, SY01-D21-8, SY01-D21-17 and SY01-D21-24 have similar blocking activity to that of the parental molecule NB22D-21-huVH2. In addition, since the cell expression level of SY01-D21-47 is not high, this clone is not selected for further validations.

### Example 18 Full-length construction of the derisked candidate clones and sample production thereof

The positive VHH candidate antibodies SY01-D21-4, SY01-D21-8, SY01-D21-17 and SY01-D21-24 obtained by screening are fused to the human IgG1 Fc fragments. The C-terminus of the positive VHH gene sequence is linked to the N-terminus of the human IgG1Fc fragment gene sequence to construct a fusion expression plasmid. The fusion expression plasmid is transformed into ExpiCHO cells to induce expression, thereby obtaining 4 VHH-Fc chimeric antibody proteins (respectively referred to as NB22D-21-4, NB22D-21-8, NB22D-21-17 and NB22D-21-24).

The antibody is expressed by using the ExpiCHO transient transfection and expression system; the culture medium is the Gibco ExpiCHO Expression Medium, A29100-01; and the transfection kit is ExpiFectamine^{™} CHO Transfection Kit, A29129. The specific method is as follows: one day prior to transfection, ExpiCHO cells are passaged, and in a 25 ml culture, 25 µg of the constructed plasmids are mixed with a transfection reagent, then added dropwise to 25 ml of ExpiCHO cell cultures, fully mixed and expressed at 37°C for 18-22 hours. Then a feed culture medium is added according to the instruction in the kit, and after feeding, the cells are cultured at 32°C. On day 5 after transfection, a second feed culture medium is added, and the cells are cultured at 32°C for 10-12 days. Then the expressed cell suspension is centrifuged at a high speed to obtain a supernatant. The obtained supernatant is filtered through 0.22 µm and then purified by using a Protein A/G affinity purification method. 100 mM glycine salt (pH 3.0) is used to elute the protein of interest followed by neutralization with 1 M Tris-HCl.

### Example 19 Verification of binding activity of the derisked candidate antibodies to human PD-L1 and monkey PD-L1

In order to evaluate 4 derisked VHH-Fc candidate antibodies, an FACS method is used to detect the binding activities thereof to cell expressed human PD-L1 and monkey PD-L1 proteins. The specific method is as follows:
1) collecting the cultured human and monkey PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the human and monkey PD-L1-CHO cells to a 96-well round-bottom plate at 100 µL/well, respectively, and centrifuging the cells at 300 g to remove the supernatant;
3) adding the gradient-diluted candidate antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding a PE-labeled anti-human IgG Fc flow cytometry antibody (Abcam, ab98596), pipetting the cells repeatedly with a multi-channel pipette and incubating the cells at 4°C for 30 minutes;
7) centrifuging the mixture at 300 g to remove the supernatant, adding the FACS buffer and resuspending the cells; and
8) repeating step 10) twice, adding the FACS buffer to the wells (200 µL/well), resuspending the cells, and performing detection by a flow cytometer (Beckman, CytoFLEX AOO-1-1102).

As shown in Table 7, the binding activities of 4 candidate molecules to the human and monkey PD-L1-CHO are compared, and the present inventors screen out 2 nanobody candidate molecules NB22D-21-4 and NB22D-21-24 which have relatively high affinities to human PD-L1 and monkey PD-L1.

**Table 7. EC50 of antibodies**

| Clone number | EC50 (µg/mL) of antibody binding to human PD-L1-CHO | EC50 (µg/mL) of antibody binding to monkey PD-L1-CHO |
|---|---|---|
| NB22D-21-huVH2 | 0.72 | 1.82 |
| NB22D-21-4 | 0.21 | 0.30 |
| NB22D-21-8 | 0.26 | 0.70 |
| NB22D-21-17 | 1.73 | 3.25 |
| NB22D-21-24 | 0.14 | 0.39 |

### Example 20 Verification of the blocking activity of the derisked candidate antibodies against PD-1

In order to evaluate the obtained VHH-Fc candidate antibodies, an FACS method is used to detect the blocking activity thereof against PD-1/PD-L1. The specific method is as follows:
1) collecting the cultured human PD-L1-CHO cells, centrifuging the collected cells at 300 g to remove the supernatant, resuspending the cells in a formulated FACS buffer and counting the cells, and adjusting the density of the cell suspension to 2×10⁶ cells/mL;
2) adding the PD-L1-CHO cells to a 96-well round-bottom plate at 100 µL/well, and centrifuging the cells at 300 g to remove the supernatant;
3) adding the gradient-diluted candidate antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) twice, and centrifuging the cell mixed solution at 300 g to remove the supernatant;
6) adding 100 µL of biotin-labeled PD-1-Fc protein diluent (1 µg/mL) to the corresponding wells, resuspending the cells and incubating the cells at 4°C for 30 minutes;
7) centrifuging the incubated cell mixed solution at 300 g to remove the supernatant, adding 200 µL of FACS buffer to the corresponding wells and resuspending the cells by using a multi-channel pipette;
8) repeating step 7) twice, and centrifuging the cell mixed solution at 300 g to remove the supernatant;
9) adding a PE-labeled streptavidin (eBioscience, 12-4317-87), pipetting the cells repeatedly with a multi-channel pipette and incubating the cells at 4°C for 30 minutes;
10) centrifuging the mixture at 300 g to remove the supernatant, adding the FACS buffer and resuspending the cells; and
11) repeating step 10) twice, adding the FACS buffer to the wells (200 µL/well), resuspending the cells, and performing detection by a flow cytometer (Beckman, CytoFLEX AOO-1-1102).

As shown in Table 8, by the FACS experiment, the present inventors verify that the 2 nanobody candidates with clone numbers NB22D-21-4 and NB22D-21-24 in Example 19 both have high blocking activities, and the blocking activities thereof are higher than that of the parental NB22D-21-huVH2 antibody.

**Table 8. IC50 of antibodies**

| Clone number | IC50 (µg/mL) |
|---|---|
| NB22D-21-huVH2 | 1.00 |
| NB22D-21-4 | 0.30 |
| NB22D-21-24 | 0.20 |

### Example 21 Specificity detection of the binding of the derisked candidate antibodies to PD-L1

In order to confirm the binding specificity of the derisked candidates to a PD-L1 protein, an FACS method is used to detect the binding specificity thereof to PD-L1 negative cells. The method is specifically divided into two parts, and the first part of the specific method is as follows:
1) collecting the cultured Jurkat and Raji cells, centrifuging the collected cells at 300 g to remove the supernatant, resuspending the cells in a formulated FACS buffer (buffer ingredients: 1*PBS+5% FBS+2% BSA) and counting the cells, and adjusting the density of the cell suspension to 2×10⁶ cells/mL;
2) adding the Jurkat and Raji cells to a 96-well round-bottom plate at 100 µL/well, respectively, and centrifuging the cells at 300 g to remove the supernatant;
3) adding the gradient-diluted candidate antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) four times, and centrifuging the cell mixed solution at 300 g to remove the supernatant;
6) adding a PE-labeled anti-human IgG Fc flow cytometry antibody (Abcam, ab98596), pipetting the cells repeatedly with a multi-channel pipette and incubating the cells at 4°C for 30 minutes;
7) centrifuging the mixture at 300 g to remove the supernatant, adding the FACS buffer and resuspending the cells; and
8) repeating step 10) twice, adding the FACS buffer to the wells (200 µL/well), resuspending the cells, and performing detection by a flow cytometer (Beckman, CytoFLEX AOO-1-1102).

As shown in Table 9 below, according to the FACS detection results, the present inventors indicate positive and negative by "+" (binding) and "-" (no binding), respectively, and Table 9 shows that for the NB22D-21-4 and NB22D-21-24 clones, the former has no non-specific binding on both cells, while the latter has relatively high non-specific binding, and the control antibody does not show non-specific binding.

**Table 9. Non-specific binding of antibodies**

| Clone/cell | Jurkat | Raji |
|---|---|---|
| NB22D-21-huVH2 | - | - |
| NB22D-21-4 | - | - |
| NB22D-21-24 | + | + |
| KN035 (control) | - | - |
| Atezolizumab (control) | - | - |

In the second part of the experiment, the present inventors use an FACS method to detect the binding specificity of the NB22D-21-4 clone to cell expressed B7 family proteins. The specific method is as follows:
1) collecting the cultured B7-H2, B7-H4 and B7-H5 over-expressing CHO cells, centrifuging the collected cells at 300 g to remove the supernatant, resuspending the cells in a formulated FACS buffer (buffer ingredients: 1*PBS+5% FBS+2% BSA) and counting the cells, and adjusting the density of the cell suspension to 2×10⁶ cells/mL;
2) adding the B7-H2, B7-H4 and B7-H5 over-expressing cells to a 96-well round-bottom plate at 100 µL/well, respectively, and centrifuging the cells at 300 g to remove the supernatant;
3) adding the gradient-diluted candidate antibody diluents and the control antibody diluents to the corresponding wells, pipetting the cells repeatedly with a multi-channel pipette and incubating the cells at 4°C for 30 minutes;
4) centrifuging the incubated cell mixed solution at 300 g to remove the supernatant, adding 200 µL FACS buffer to the corresponding wells and resuspending the cells by using a multi-channel pipette;
5) repeating step 4) four times, and centrifuging the cell mixed solution at 300 g to remove the supernatant;
6) adding a PE-labeled anti-human IgG Fc flow cytometry antibody (Abcam, ab98596), pipetting the cells repeatedly with a multi-channel pipette and incubating the cells at 4°C for 30 minutes;
7) centrifuging the mixture at 300 g to remove the supernatant, adding the FACS buffer and resuspending the cells; and
8) repeating step 10) twice, adding the FACS buffer to the wells (200 µL/well), resuspending the cells, and performing detection by a flow cytometer (Beckman, CytoFLEX AOO-1-1102).

As shown in Table 10 below, according to the FACS detection results, the present inventors indicate positive and negative by "+" (binding) and "-" (no binding), respectively, and Table 10 shows that NB22D-21-4 does not have non-specific binding on a variety of cells, and the control antibody does not show non-specific binding.

**Table 10. Specificity of antibodies to B7 family proteins**

| Clone/cell | B7-H2 | B7-H3 | B7-H4 | B7-H5 |
|---|---|---|---|---|
| NB22D-21-huVH2 | - | - | - | - |
| NB22D-21-4 | - | - | - | - |
| KN035 (control) | - | - | - | - |

### Example 22 Verification of in vitro MLR activity of the NB22D-21-4 antibody

In order to confirm the ability of the candidate antibody to stimulate T cell activation *in vitro,* the present inventors establish a mixed lymphocyte reaction assay *in vitro.* The specific method is as follows:
CD14-positive monocytes and CD4-positive T cells are sorted *in vitro* by using a Miltenyibiotec sorting kit (Miltenyibiotec, 130-050-201). The monocytes are induced into dendritic cells (DCs) by *in vitro* induction culture with 100 ng/mL IL-4 and 100 ng/mL GM-CSF for 7 days. Then the CD4-positive T cells and DCs are mixed at a cell number ratio of 10:1, and the gradient-diluted candidate antibody and the control antibody are added to the cells and cultured in a cell incubator at 37°C for 5 days. After 72 hours of culture, the cell supernatant is taken and diluted with PBS, so as to detect the secretion level of IL-2 in the culture supernatant by using an IL-2 ELISA kit; and after 5 days of culture, the cell supernatant is taken and diluted with PBS, so as to detect the secretion level of IFN-γ in the culture supernatant by using an IFN-γ ELISA kit.

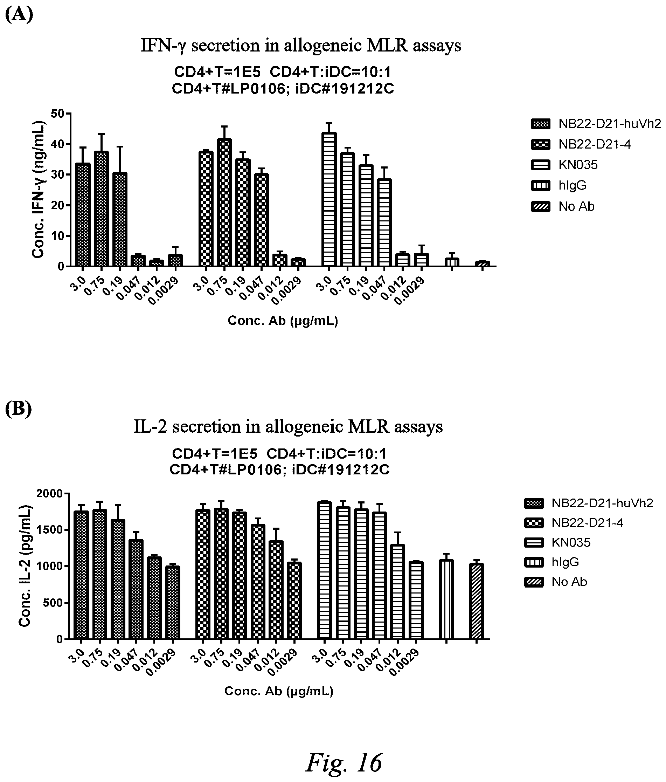

By detecting the secretion levels of IFN-γ and IL-2, the present inventors obtain the verification results of mixed lymphocyte reaction assay of the candidate antibody, which results are shown in Figure 16. Figure 16 shows that the nanobody candidate molecule NB22D-21-4 of the present invention can activate the T cell immune response.

### Example 23 Affinity maturation of the candidate antibodies

In order to further improve the affinity and blocking activity of the candidate antibodies in binding to a mouse PD-L1 antigen, we perform affinity maturation design on humanized candidate antibodies.

This round of affinity maturation is performed by using a single-site saturation mutagenesis method; a Trim primer technique is used to directionally design primers for the CDR regions, thereby constructing an affinity-matured antibody library; and subsequently a phage display technique is used to screen the affinity-matured library. The affinity maturation library is subjected to panning and preliminary screening, respectively by using mouse antigens; monoclones are obtained for the subsequent construction and expression of full-length antibodies; and the candidate clones are tested for affinity, human-mouse cross reactivity and blocking activity by FACS. By means of screening, we obtain 5 clones with better activity than that of the parental, namely NB22D-21-45-106, NB22D-21-123, NB22D-21-154, NB22D-21-94 and NB22D-21-109, respectively.

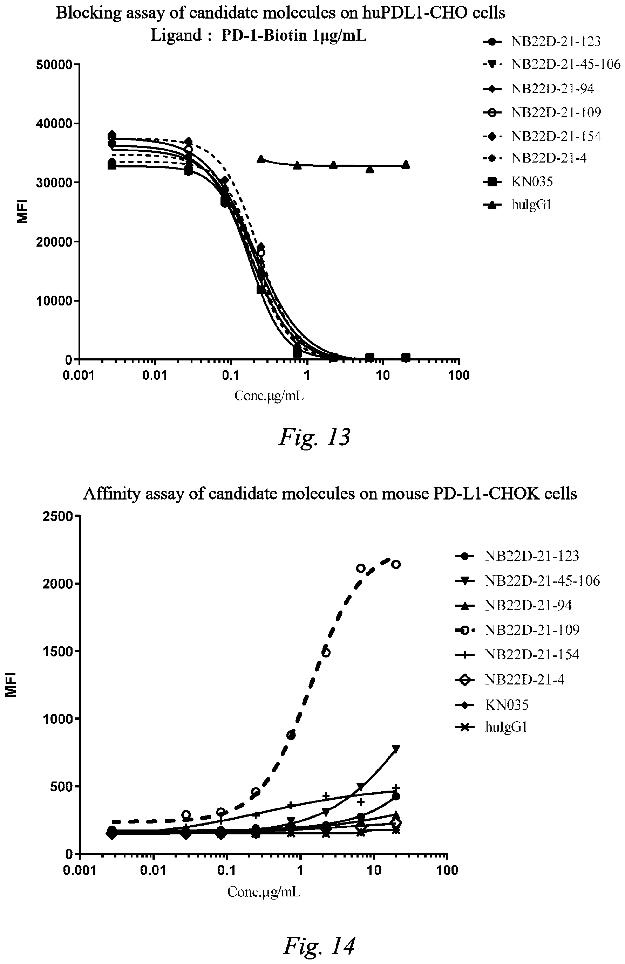

The specific experimental results are shown in Figure 12, Figure 13 and Figure 14. The results showed that the five molecules NB22D-21-45-106, NB22D-21-123, NB22D-21-154, NB22D-21-94 and NB22D-21-109 have the similar or better binding activity and blocking activity to or than that of the control antibody NB22D21-4, and have better mouse cross activity than that of the parental NB22D-21-huVH2. The experimental results of Example 15 and Example 17 showed that the NB22D21-4 molecule has better binding activity and blocking activity than that of the parental antibody NB22D-21, and it can be deduced that the 5 molecules obtained by affinity maturation have highly similar or better binding and blocking activity to or than that of the parental antibody NB22D-21, and have better mouse cross activity than that of the parental antibody NB22D-21 and the control antibody KN035.

A experienced specialist in the field will further appreciate that the present invention may be embodied in other specific forms without departing from the spirit or central features thereof. In that the foregoing description of the present invention discloses only exemplary embodiments thereof, it is to be understood that other variations are contemplated as being within the scope of the present invention. Accordingly, the present invention is not limited to the particular embodiments that have been described in detail herein. Rather, reference should be made to the appended claims as indicative of the scope and content of the invention.

## Claims

1. An isolated PD-L1 binding molecule, which specifically binds to PD-L1 and comprises a heavy chain variable region, wherein the heavy chain variable region comprises:
(i) a CDR1 comprising the amino acid sequence of SEQ ID NO: 1;
a CDR1 comprising an amino acid sequence having at least 80%, 85%, 90%, 95% or 99% identity to SEQ ID NO: 1; or
a CDR1 comprising an amino acid sequence which differs from SEQ ID NO: 1 by no more than 2 (e.g., 0, 1 and 2) amino acid additions, deletions and/or substitutions;
(ii) a CDR2 comprising the amino acid sequence of SEQ ID NO: 2;
a CDR2 comprising an amino acid sequence having at least 80%, 85%, 90%, 95% or 99% identity to SEQ ID NO: 2; or
a CDR2 comprising an amino acid sequence which differs from SEQ ID NO: 2 by no more than 2 (e.g., 0, 1, and 2) amino acid additions, deletions and/or substitutions;
and
(iii) a CDR3 comprising the amino acid sequence of SEQ ID NO: 3 or 12;
a CDR3 comprising an amino acid sequence having at least 80%, 85%, 90%, 95% or 99% identity to SEQ ID NO: 3 or 12; or
a CDR3 comprising an amino acid sequence which differs from SEQ ID NO: 3 or 12 by no more than 2 (e.g., 0, 1, and 2) amino acid additions, deletions and/or substitutions.

2. The isolated PD-L1 binding molecule according to claim 1, wherein the heavy chain variable region comprises:
(i) a CDR1 as shown in formula RTDSNIX₁GMH, wherein X₁ is H, F or N;
(ii) a CDR2 as shown in formula TIFIDX₂NTX₃, wherein X₂ is G, L or A, and X₃ is I or L; and
(iii) a CDR3 as shown in formula DVSGYGRX₄, wherein X₄ is A or Y.

3. The isolated PD-L1 binding molecule according to claim 1, wherein the heavy chain variable region comprises:
(i) a CDR1 comprising or consisting of SEQ ID NO: 1;
(ii) a CDR2 comprising or consisting of SEQ ID NO: 2; and
(iii) a CDR3 comprising or consisting of SEQ ID NO: 3.

4. The isolated PD-L1 binding molecule according to claim 1, wherein the heavy chain variable region comprises:
(i) a CDR1 comprising or consisting of SEQ ID NO: 4;
(ii) a CDR2 comprising or consisting of SEQ ID NO: 5; and
(iii) a CDR3 comprising or consisting of SEQ ID NO: 3.

5. The isolated PD-L1 binding molecule according to claim 1, wherein the heavy chain variable region comprises:
(i) a CDR1 comprising or consisting of SEQ ID NO: 6;
(ii) a CDR2 comprising or consisting of SEQ ID NO: 7; and
(iii) a CDR3 comprising or consisting of SEQ ID NO: 3.

6. The isolated PD-L1 binding molecule according to claim 1, wherein the heavy chain variable region comprises:
(i) a CDR1 comprising or consisting of SEQ ID NO: 1;
(ii) a CDR2 comprising or consisting of SEQ ID NO: 8; and
(iii) a CDR3 comprising or consisting of SEQ ID NO: 9.

7. The isolated PD-L1 binding molecule according to claim 1, wherein the heavy chain variable region comprises:
(i) a CDR1 comprising or consisting of SEQ ID NO: 10;
(ii) a CDR2 comprising or consisting of SEQ ID NO: 11; and
(iii) a CDR3 comprising or consisting of SEQ ID NO: 12.

8. The isolated PD-L1 binding molecule according to claim 1, wherein the heavy chain variable region comprises:
(i) a CDR1 comprising or consisting of SEQ ID NO: 13;
(ii) a CDR2 comprising or consisting of SEQ ID NO: 5; and
(iii) a CDR3 comprising or consisting of SEQ ID NO: 3.

9. The isolated PD-L1 binding molecule according to claim 1, wherein the heavy chain variable region comprises:
(i) a CDR1 comprising or consisting of SEQ ID NO: 14;
(ii) a CDR2 comprising or consisting of SEQ ID NO: 15; and
(iii) a CDR3 comprising or consisting of SEQ ID NO: 3.

10. The isolated PD-L1 binding molecule according to claim 1, wherein the heavy chain variable region comprises:
(i) a CDR1 comprising or consisting of SEQ ID NO: 16;
(ii) a CDR2 comprising or consisting of SEQ ID NO: 17; and
(iii) a CDR3 comprising or consisting of SEQ ID NO: 3.

11. The isolated PD-L1 binding molecule according to claim 1, wherein the heavy chain variable region comprises:
(i) a CDR1 comprising or consisting of SEQ ID NO: 18;
(ii) a CDR2 comprising or consisting of SEQ ID NO: 19; and
(iii) a CDR3 comprising or consisting of SEQ ID NO: 3.

12. The isolated PD-L1 binding molecule according to claim 1, wherein the heavy chain variable region comprises:
(i) a CDR1 comprising or consisting of SEQ ID NO: 20;
(ii) a CDR2 comprising or consisting of SEQ ID NO: 5; and
(iii) a CDR3 comprising or consisting of SEQ ID NO: 3.

13. An isolated PD-L1 binding molecule, which specifically binds to PD-L1 and comprises a heavy chain variable region, wherein the heavy chain variable region comprises:
(i) a CDR1 comprising or consisting of SEQ ID NO: 21;
(ii) a CDR2 comprising or consisting of SEQ ID NO: 22; and
(iii) a CDR3 comprising or consisting of SEQ ID NO: 23.

14. An isolated PD-L1 binding molecule, which specifically binds to PD-L1 and comprises a heavy chain variable region, wherein the heavy chain variable region comprises:
(i) a CDR1 comprising or consisting of SEQ ID NO: 24;
(ii) a CDR2 comprising or consisting of SEQ ID NO: 25; and
(iii) a CDR3 comprising or consisting of SEQ ID NO: 3.

15. An isolated PD-L1 binding molecule, which specifically binds to PD-L1 and comprises a heavy chain variable region, wherein the heavy chain variable region comprises:
(i) a CDR1 comprising or consisting of SEQ ID NO: 26;
(ii) a CDR2 comprising or consisting of SEQ ID NO: 27; and
(iii) a CDR3 comprising or consisting of SEQ ID NO: 23.

16. An isolated PD-L1 binding molecule, which specifically binds to PD-L1 and comprises a heavy chain variable region, wherein the heavy chain variable region comprises:
(i) a CDR1 comprising or consisting of SEQ ID NO: 28;
(ii) a CDR2 comprising or consisting of SEQ ID NO: 29; and
(iii) a CDR3 comprising or consisting of SEQ ID NO: 23.

17. An isolated PD-L1 binding molecule, which specifically binds to PD-L1 and comprises a heavy chain variable region, wherein the heavy chain variable region comprises:
(i) a CDR1 comprising or consisting of SEQ ID NO: 30;
(ii) a CDR2 comprising or consisting of SEQ ID NO: 31; and
(iii) a CDR3 comprising or consisting of SEQ ID NO: 23.

18. The isolated PD-L1 binding molecule according to any one of claims 1-17, wherein the heavy chain variable region further comprises an FR region comprising FR1, FR2, FR3 and FR4, and the FR1, FR2, FR3 and FR4 and CDR1, CDR2 and CDR3 are arranged alternately on the heavy chain variable region to form a structure of FR1-CDR1-FR2-CDR2-FR3-CDR3-FR4 from N-terminus to C-terminus.

19. The isolated PD-L1 binding molecule according to claim 18, wherein the FR region comprises:
(a) an FR1 comprising the amino acid sequence of SEQ ID NO: 53;
an FR1 comprising an amino acid sequence having at least 90%, 95% or 99% identity to SEQ ID NO: 53; or
an FR1 comprising an amino acid sequence which differs from SEQ ID NO: 53 by no more than 2 (e.g., 0, 1 and 2) amino acid additions, deletions and/or substitutions of amino acids;
(b) an FR2 comprising the amino acid sequence of SEQ ID NO: 54;
an FR2 comprising an amino acid sequence having at least 90%, 95% or 99% identity to SEQ ID NO: 54; or
an FR2 comprising an amino acid sequence which differs from SEQ ID NO: 54 by no more than 2 (e.g., 0, 1 and 2) amino acid additions, deletions and/or substitutions of amino acids;
(c) an FR3 comprising the amino acid sequence of SEQ ID NO: 55;
an FR3 comprising an amino acid sequence having at least 90%, 95% or 99% identity to SEQ ID NO: 55; or
an FR3 comprising an amino acid sequence which differs from SEQ ID NO: 55 by no more than 2 (e.g., 0, 1 and 2) amino acid additions, deletions and/or substitutions of amino acids;
and
(d) an FR4 comprising the amino acid sequence of SEQ ID NO: 56;
an FR4 comprising an amino acid sequence having at least 90%, 95% or 99% identity to SEQ ID NO: 56; or
an FR4 comprising an amino acid sequence which differs from SEQ ID NO: 56 by no more than 2 (e.g., 0, 1, and 2) amino acid additions, deletions and/or substitutions.

20. The isolated PD-L1 binding molecule according to claim 18, wherein the FR region comprises:
(a) an FR1 comprising the amino acid sequence of SEQ ID NO: 57;
an FR1 comprising an amino acid sequence having at least 90%, 95% or 99% identity to SEQ ID NO: 57; or
an FR1 comprising an amino acid sequence which differs from SEQ ID NO: 57 by no more than 2 (e.g., 0, 1 and 2) amino acid additions, deletions and/or substitutions;
(b) an FR2 comprising the amino acid sequence of SEQ ID NO: 58;
an FR2 comprising an amino acid sequence having at least 90%, 95% or 99% identity to SEQ ID NO: 58; or
an FR2 comprising an amino acid sequence which differs from SEQ ID NO: 58 by no more than 2 (e.g., 0, 1 and 2) amino acid additions, deletions and/or substitutions;
(c) an FR3 comprising the amino acid sequence of SEQ ID NO: 59;
an FR3 comprising an amino acid sequence having at least 90%, 95% or 99% identity to SEQ ID NO: 59; or
an FR3 comprising an amino acid sequence which differs from SEQ ID NO: 59 by no more than 2 (e.g., 0, 1 and 2) amino acid additions, deletions and/or substitutions;
and
(d) an FR4 comprising the amino acid sequence of SEQ ID NO: 60;
an FR4 comprising an amino acid sequence having at least 90%, 95% or 99% identity to SEQ ID NO: 60; or
an FR4 comprising an amino acid sequence which differs from SEQ ID NO: 60 by no more than 2 (e.g., 0, 1, and 2) amino acid additions, deletions and/or substitutions.

21. The isolated PD-L1 binding molecule according to claim 18, wherein the FR region comprises:
(a) an FR1 comprising the amino acid sequence of SEQ ID NO: 61;
an FR1 comprising an amino acid sequence having at least 90%, 95% or 99% identity to SEQ ID NO: 61; or
an FR1 comprising an amino acid sequence which differs from SEQ ID NO: 61 by no more than 2 (e.g., 0, 1 and 2) amino acid additions, deletions and/or substitutions;
(b) an FR2 comprising the amino acid sequence of SEQ ID NO: 58;
an FR2 comprising an amino acid sequence having at least 90%, 95% or 99% identity to SEQ ID NO: 58; or
an FR2 comprising an amino acid sequence which differs from SEQ ID NO: 58 by no more than 2 (e.g., 0, 1 and 2) amino acid additions, deletions and/or substitutions;
(c) an FR3 comprising the amino acid sequence of SEQ ID NO: 59;
an FR3 comprising an amino acid sequence having at least 90%, 95% or 99% identity to SEQ ID NO: 59; or
an FR3 comprising an amino acid sequence which differs from SEQ ID NO: 59 by no more than 2 (e.g., 0, 1 and 2) amino acid additions, deletions and/or substitutions;
and
(d) an FR4 comprising the amino acid sequence of SEQ ID NO: 60;
an FR4 comprising an amino acid sequence having at least 90%, 95% or 99% identity to SEQ ID NO: 60; or
an FR4 comprising an amino acid sequence which differs from SEQ ID NO: 60 by no more than 2 (e.g., 0, 1, and 2) amino acid additions, deletions and/or substitutions.

22. The isolated PD-L1 binding molecule according to any one of claims 1-17, wherein the heavy chain variable region comprises or consists of an amino acid sequence of any one of SEQ ID NOs: 36-52.

23. The isolated PD-L1 binding molecule according to any one of claims 1-17, wherein the heavy chain variable region comprises an amino acid sequence having at least 80%, 85%, 90%, 95% or 99% identity to an amino acid sequence of any one of SEQ ID NOs: 36-52 and retaining the ability to specifically bind to PD-L1.

24. The isolated PD-L1 binding molecule according to any one of claims 1-17, wherein the heavy chain variable region comprises an amino acid sequence having one or more amino acid additions, deletions and/or substitutions (e.g., conservative substitutions) compared to an amino acid sequence of any one of SEQ ID NOs: 36-52 and retaining the ability to specifically bind to PD-L1, wherein the one or more amino acid additions, deletions and/or substitutions (e.g., conservative substitutions) are no more than five, preferably no more than three.

25. The isolated PD-L1 binding molecule according to any one of claims 1-17, wherein the PD-L1 binding molecule is a camelized antibody, a humanized antibody, an affinity-matured antibody or a druggability-modified molecule.

26. The isolated PD-L1 binding molecule according to any one of claims 1-17, wherein the PD-L1 binding molecule is a single-domain antibody or an antigen-binding fragment thereof.

27. The isolated PD-L1 binding molecule according to any one of claims 1-17, wherein the heavy chain variable region is fused to an additional molecule, and the additional molecule is selected from an Fc domain of an immunoglobulin (e.g., IgG), an antibody, an antigen-binding fragment of the antibody, an antibody-drug conjugate, an antibody-like molecule, an antigen-binding fragment of the antibody-like molecule or a fluorescent protein.

28. The isolated PD-L1 binding molecule according to claim 27, wherein the heavy chain variable region is fused to an Fc domain of human IgG (e.g., human IgG1or human IgG4).

29. An isolated nucleic acid molecule, comprising a nucleotide sequence encoding the isolated PD-L1 binding molecule according to any one of claims 1-17.

30. A vector, comprising the nucleic acid molecule according to claim 29.

31. A host cell, comprising the vector according to claim 30.

32. A pharmaceutical composition, comprising an effective amount of the PD-L1 binding molecule according to any one of claims 1-17 and a pharmaceutically acceptable carrier.

33. A method for preparing the PD-L1 binding molecule according to any one of claims 1-17, wherein the method comprises the following steps:
- expressing the nucleotide sequence encoding the PD-L1 binding molecule according to any one of claims 1-17 in a host cell to produce the PD-L1 binding molecule; and
- isolating the PD-L1 binding molecule from the host cell.

34. A method for preventing or treating a disease associated with PD-L1 in a subject, the method comprising administering a therapeutically effective amount of the PD-L1 binding molecule according to any one of claims 1-17 to the subject.

35. The method according to claim 34, wherein the subject is a mouse or a human, preferably a human.

36. The method according to claim 34, wherein the disease associated with PD-L1 is selected from renal cell carcinoma, non-small cell lung cancer, bladder cancer, urothelial carcinoma, and microsatellite unstable solid tumor.

37. A kit for preventing or treating a disease associated with PD-L1 in a subject, comprising a container, wherein the container comprises at least one PD-L1 binding molecule according to any one of claims 1-28.
